# EUROPEAN PATENT SPECIFICATION

(11) **EP 0 702 564 B1**
(45) Date of publication and mention of the grant of the patent: **01.03.2000**
(21) Application number: 94917176.3
(22) Date of filing: 02.06.1994
(51) Int. Cl.: A61K 39/104, C12N 15/31

(54) **NOVEL ATTENUATED PSEUDOMONAS AERUGINOSA STRAINS**
NEUARTIGE ABGESCHWÄCHTE STÄMME VON PSEUDOMONAS AERUGINOSA
NOUVELLE SOUCHE DE PSEUDOMONAS AERUGINOSA ATTENUEE

(30) Priority: 07.06.1993 KR 9310273; 07.06.1993 KR 9310281
(43) Date of publication of application: 27.03.1996
(73) Proprietor: Cheil Jedang Corporation, Seoul (KR)
(72) Inventor: KIM, Hyun, Su, Kangdong-gu Seoul 134-012 (KR); PARK, Wan, Je, Suwon-shi Kyunggi-do 441-370 (KR); MOON, Moo, Sang, Songpa-gu Seoul 138-160 (KR); YOO, Wang, Don, Dongjak-gu Seoul 156-020 (KR); NOH, Kap, Soo, Dognjak-gu Seoul 156-034 (KR); LEE, Dong, Eok, Dongdaemoon-gu Seoul 131-022 (KR); HA, Suk, Hoon, Kangnam-gu Seoul 135-092 (KR); YOO, Ree, An, Kwachun-shi Kyunggi-do 427-040 (KR); LEE, Nam, Joong, Seongbuk-gu Seoul 136-103 (KR); CHO, Yang, Je, Dongjak-gu Seoul 156-090 (KR); HONG, Sun, Pyo, Seongbuk-gu Seoul 136-071 (KR); KIM, Je, Hak, Kwachun-shi 427-050 Kyunggi-do (KR); KIM, Dal, Hyun, Suwon-shi Kyunggi-do 440-240 (KR); KIM, Young, Gi, Seoul 131-202 (KR)
(74) Representative: Rinuy, Santarelli
(86) International application number: KR9400062
(87) International publication number: WO9428928

(56) References cited:
- EP-A- 0 357 024
- US-A- 3 928 565
- US-A- 3 987 164

## Description

### Field of the Invention

The present invention relates to novel attenuated Pseudomonas aeruginosa strains, a prophylactic vaccine and a therapeutic agent for a Pseudomonas aeruginosa infection, and a method for preparation thereof. More specifically, the present invention relates to novel attenuated Pseudomonas aeruginosa strains which are obtained by isolating Pseudomonas aeruginosa in a pure state according to Fisher-Devlin immunotype and then repeatedly purifying the isolated strain by sequential passes through mice, a prophylactic vaccine prepared from, and therapeutic immunoglobulins induced by cell wall proteins separated from the attenuated Pseudomonas aeruginosa strain, and a method for preparation thereof.

### Description of the Prior Art

Pseudomonas aeruginosa is a motile gram-negative rod, approximately 0.5 um x 1.5-3.0 um, having a single flagellum and occurs widely in soil, water, sewage and human intestine (Mol. Microbiol. 4, 1069-1075, 1990). Pseudomonas aeruginosa is a pathogenic strain causative of inveterate infections such as septicemia, generalized infection, chronic respiratory tract infection, cystic fibrosis of the pancreas, etc. The septicemia caused by Pseudomonas aeruginosa is a disease resulting from either the invasion of the microorganism itself or the secretion of its toxic constituents into the blood of patients who have lowered resistance due to surgery, laceration, trauma and the like. The presence of the toxin causes shock with high fever, reduced blood pressure, and other symptoms, which ultimately may lead to death. Furthermore, since Pseudomonas aeruginosa has been detected in urinary tract infections, interest in Pseudomonas aeruginosa has greatly increased. Accordingly, development of a medicinal agent(s) capable of effectively preventing or treating inveterate suppurative diseases, such as septicemia and urinary tract infections, caused by Pseudomonas aeruginosa is urgently needed. However, Pseudomonas aeruginosa strains are resistant to most antibiotic substances and an effective preventive or therapeutic agent for Pseudomonas aeruginosa infections has not been developed up to date. Thus, the virulence of Pseudomonas aeruginosa infections has increased over time.

Pseudomonas aeruginosa strains can be classified in various ways. One such classification system classifies the strains into seven (7) types according to Fisher immunotype. Another system is based on the O-antigen group as proposed by Terada. Still another system is the International Antigenic Typing Scheme (IATS) classification system. In view of classification systems, the Pseudomonas aeruginosa strains most frequently occurring in Pseudomonas aeruginosa-infected patients are: 5/2a, 2c, 3, 7/3a, 3c, 1/4a, 4b, 6/5a, 5b, 4/6, 2/7a, 7b, 7c, /10a, /13, /12, /11 and 3,7/3d, 3e types according to Fisher immunotype/O-serotype, with 3, 7, 2 and 1 types as Fisher immunotype (corresponding to 3a, 3c, 3d, 3e, 7a, 7b, 7c, 4a and 4b as O-serotype) being mainly present.

One method of treatment for Pseudomonas aeruginosa infections neutralizes the Pseudomonas aeruginosa toxins with antitoxins. However, the antitoxin is a therapeutic agent, which is only beneficial in patients already suffering from septicemia, and has no prophylactic effect. Further, the antitoxin as presently available is very expensive and its use is limited. In addition, the most significant disadvantage accompanying the use of the antitoxin is the relatively low cure rate and the accompanying severe side effects.

One method under study to avoid the disadvantages associated with the antitoxin uses a common antigen for the prevention and treatment of Pseudomonas aeruginosa infections. Methods under study for obtaining the common antigen can be generally classified into two groups. The first method relates to the use of a common antigen separated and purified from Pseudomonas aeruginosa strains having different immunotypes as a prophylactic vaccine antigen against Pseudomonas aeruginosa infections [Japan, J. Exp. Med. 45, 355-359, 1975]. The second method relates to the use of common antigen mass produced by utilizing a genetic engineering technique in which a gene coding for the desired antigen is isolated and inserted into a suitable vector to obtain a recombinant vector and then the suitable host is transformed with the resulting recombinant vector and incubated to express the desired antigen.

Although development of a prophylactic vaccine using a common antigen is a very effective method in theory, at present the progress of study relating to this method indicates that this method has many problems which remain unsolved. The major disadvantage is that the common antigen cannot prevent all kinds of Pseudomonas aeruginosa having different immunotypes and therefore its effectiveness is extremely low. Such low effectiveness suggests that the presence of another unknown major antigen, in addition to the common antigen, may provoke an effective prophylactic effect.

Another method for treating Pseudomonas aeruginosa infection is the administration of antibiotics or chemo-therapeutic agents having broad-spectrum selectivity for the Pseudomonas aeruginosa strain. However, since there are numerous Pseudomonas aeruginosa strains and they generally have a very high degree of drug resistance, many patients have succumb to Pseudomonas aeruginosa strains which cannot be effectively treated by antibiotics.

In addition, a method using therapeutic immunoglobulin has been developed. However, such immunoglobulin exhibits no or little therapeutic effect on all Pseudomonas aeruginosa infections and thus has been used only for very limited types of Pseudomonas aeruginosa infections. This is mainly due to the immunoglobulin being prepared according to a method for preparing a polyclonal antibody to a certain microorganism and therefore cannot commonly act on the numerous Pseudomonas aeruginosa strains.

Attempts have been made to develop an inexpensive therapeutic agent with mouse monoclonal antibodies [J. Inf. Dis. 152, 1290-1299, 1985] or human monoclonal antibodies [FEMS Microbiol. Immunol. 64, 263-268, 1990] against Pseudomonas aeruginosa. Here, cell lines can be selected to produce the most effective neutralizing antibodies from a cell bank with cell fusion technique and then a cell line can be used as starting materials to produce the desired antibody on an industrial scale. However, this method aims at the treatment of Pseudomonas aeruginosa infection via antibody production but not at prophylactic vaccines. In addition, this method has a disadvantage that since all infections are caused by different Pseudomonas aeruginosa strains with different serological and immunological types, they all cannot be treated with only one kind of monoclonal antibody. That is, monoclonal antibody therapy cannot be effectively utilized to treat all the Pseudomonas aeruginosa infected patients.

To broaden effective treatment by this method, concurrent administration of several kinds of antibodies on the basis of their investigated cross-reactivity has been developed. Here, blood is collected from Pseudomonas aeruginosa infected patients and examined to identify the serological and/or immunological type of the infecting Pseudomonas aeruginosa strains. Then monoclonal antibodies suitable to the identified type are administered to the patient. However, this method requires a lot of time and therefore is unavailable to patients whose condition necessitates immediate treatment.

In the prior art, the use of cell wall proteins separated from Pseudomonas aeruginosa strains as an antigen for a vaccine has been proposed [see Vaccine, Vol. 7, "Experimental studies on the protective efficacy of a Pseudomonas aeruginosa vaccine", 1989]. However, the method proposed in the above reference uses four kinds of general Pseudomonas aeruginosa strains, i.e. NN 170041, NN 170015, NN 868 and NN 170046, which are not attenuated and, therefore, a problem concerned with the toxicities of the Pseudomonas aeruginosa strains themselves is present. Further, since in preparing a protein-component vaccine from such Pseudomonas aeruginosa strains, cells may be destroyed releasing into the medium foreign nucleic acids and toxic high molecular substances, lipopolysaccharides (LPS), which are present in cytoplasm. These substances are then incorporated into the resulting vaccine as impurities increasing the care required in administering the vaccine and possibly restricting its use.

It is also known from US 3,987, 164 to use a cell wall protein component of pseudomonas aeruginosa called CWP for the prevention of pseudomonas aeruginosa infections. However, the method proposed in this reference also uses pseudomonas aeruginosa strains which are not attenuated.

### DISCLOSURE OF INVENTION

Thus, the present inventors have extensively conducted research to find a means capable of effectively inducing antibodies against Pseudomonas aeruginosa and which manifests good immunogenic (i.e. antigenic) activity with extremely remote risk due to inherent toxicities of Pseudomonas aeruginosa strains themselves. As a result, the present inventors confirmed that when Pseudomonas aeruginosa strains isolated from Pseudomonas aeruginosa infected patients are classified into seven (7) species based on their immunotypes and then each pure strain is attenuated by passing the organism several times through a suitable animal host novel, safe and attenuated Pseudomonas aeruginosa strains can be obtained. These strains have cell wall proteins not only which are useful in preparing a vaccine for prophylaxis against Pseudomonas aeruginosa infections but also which can be used as an antibody inducer to produce immunoglobulins for Pseudomonas aeruginosa infection in the animal body. The resulting immunoglobulins exhibit superior therapeutic effect on various Pseudomonas aeruginosa infections, including severe cases, according to the present invention.

Therefore, it is an object of the present invention to provide a novel and safe attenuated Pseudomonas aeruginosa strain having cell wall proteins which exhibit antigenicity without the toxicity of the Pseudomonas aeruginosa strain itself.

It is a further object of the present invention to provide a vaccine for inducing an immune response to prevent subsequent disease caused by Pseudomonas aeruginosa in a patient receiving the vaccine.

It is a further object of the present invention to provide a method for preparing the vaccine.

It is an another object of the present invention to provide a therapeutic agent for the treatment of Pseudomonas aeruginosa infection which comprises at least one immunoglobulin for Pseudomonas aeruginosa strain.

It is a further object of the present invention to provide a method for preparing the immunoglobulin.

The foregoing has outlined some of the more pertinent objects of the present invention. These objects should be construed to be merely illustrative of some of the more pertinent features and applications of the invention. Many other beneficial results can be obtained by applying the disclosed invention in a different manner or modifying the invention within the scope of the disclosure. Accordingly, other objects and a more thorough understanding of the invention may be had by referring to the summary of the invention and the detailed description describing the preferred embodiment in addition to the scope of the invention defined by the claims taken in conjunction with the accompanying drawings.

### SUMMARY OF INVENTION

One embodiment of the present invention relates to novel, safe and effective attenuated Pseudomonas aeruginosa strains having cell wall proteins which manifest antigenicity without the toxicity of the Pseudomonas aeruginosa strain itself. Specifically, the present invention relates to safe attenuated Pseudomonas aeruginosa strains which are obtained by isolating Pseudomonas aeruginosa strains from patients infected with Pseudomonas aeruginosa, classifying the isolated microorganism strains into seven (7) types of species according to the Fisher-Devlin Immunotype, selecting a single representative strain for each immunotype, purifying the selected strain via repeat injection/isolation/re-injection steps into experimental animals and selecting the most attenuated strain, for each of seven (7) types of Pseudomonas aeruginosa strains.

A further embodiment of the present invention is directed to a novel vaccine and the process for the preparation of the vaccine for prophylaxis against Pseudomonas aeruginosa infection. The vaccine is prepared by separating cell wall proteins in a substantially pure state from each of seven (7) types of safe attenuated Pseudomonas aeruginosa strains as described below and further purifying the separated cell wall proteins and then, preferably, combining the purified cell wall proteins derived from at least 3 types of attenuated Pseudomonas aeruginosa strains. Preferably equivalent amounts of cell wall proteins from each of the 3 types are used in preparing the vaccine according to the present invention.

Another embodiment of the present invention is a therapeutic agent for Pseudomonas aeruginosa infection, which contains at least one immunoglobulin produced by cultivating at least one safe attenuated Pseudomonas aeruginosa strain, separating the cell wall protein, purifying the separated cell wall protein and then injecting the pure cell wall protein as an antibody inducer into experimental animals to induce the immunoglobulin, and a method for preparing the therapeutic agent.

The more pertinent and important features of the present invention have been outlined above in order that the detailed description of the invention which follows will be better understood and that the present contribution to the art can be fully appreciated. Additional features of the invention described hereinafter form the subject of the claims of the invention. Those skilled in the art can appreciate that the conception and the specific embodiment disclosed herein may be readily utilized as a basis for modifying or designing other structures for carrying out the same purposes of the present invention. Further, those skilled in the art can realize that such equivalent constructions do not depart from the invention as set forth in the claims.

### BRIEF DESCRIPTION OF THE DRAWINGS

For a thorough understanding of the nature and objects of the invention, reference should be had to the following detailed description taken in connection with the accompanying drawings in which:
Fig. 1 shows the result of SDS-PAGE of purified cell wall proteins separated from attenuated Pseudomonas aeruginosa strains according to the present invention; and
Fig. 2 is a graph which represents the weight change in male mouse into which the cell wall protein of attenuated Pseudomonas aeruginosa strains according to the present invention are intravenously injected.

### BEST MODE FOR CARRYING OUT THE INVENTION

Fisher-Devlin Immunotypes of Pseudomonas aeruginosa are classified into seven types, type 1 through type 7. In the present invention, Pseudomonas aeruginosa strains having Fisher immunotypes 1 to 7 are selected as strains for attenuation, on the basis of the fact that they are detected in the blood of the major portion, i.e. 90 to 95% or more, of Pseudomonas aeruginosa infected patients. Particularly, among the seven (7) types of Pseudomonas aeruginosa type 1 and type 3 are the most frequently detected strains in Pseudomonas aeruginosa infected patients. However, the teaching of the present invention may be used against any of the strains of Pseudomonas aeruginosa to provide protection or treatment.

Safe attenuated Pseudomonas aeruginosa strains according to the present invention can be obtained by isolating each of seven types of Pseudomonas aeruginosa strains in a pure state from the blood of patients who are determined as having Pseudomonas aeruginosa infection and then attenuating the isolated strains with repeated purifying procedures. The safe attenuated Pseudomonas aeruginosa strains thus obtained are all seven (7) types and designated CFCPA 10142 (Fisher type 1), CFCPA 20215 (Fisher type 2), CFCPA 30720 (Fisher type 3), CFCPA 40057 (Fisher type 4), CFCPA 50243 (Fisher type 5), CFCPA 60534 (Fisher type 6) and CFCPA 70018 (Fisher type 7), respectively. Here, CFCPA is an acronym of "Cheil Food and Chemical Pseudomonas aeruginosa", which is composed of Cheil Food and Chemicals Inc., in which the inventors of the present invention are employed, and the name of the subject strain, Pseudomonas aeruginosa.

The attenuated Pseudomonas aeruginosa strains obtained by repeated purifying procedures as described above have phenotypes and microbiological properties substantially identical to those of the corresponding parent strains but show new properties useful in the preparation of a vaccine antigen for prophylaxis against Pseudomonas aeruginosa infection rather than any pathogenicity which is present in the parent strains prior to attenuation. Accordingly, the attenuated Pseudomonas aeruginosa strains according to the present invention are regarded as novel microorganism strains. Therefore, these strains were deposited at the Korean Culture Center of Microorganisms in the Korean Federation of Culture Collections, hereafter KCCM, as an international depository authority under Budapest Treaty on May 12, 1993 under accession numbers of KCCM 10029 for CFCPA 10142, KCCM 10030 for CFCPA 20215, KCCM 10031 for CFCPA 30720, KCCM 10032 for CFCPA 40057, KCCM 10033 for CFCPA 50243, KCCM 10034 for CFCPA 60534 and KCCM 10035 for CFCPA 70018.

The purifying procedures for producing the attenuated Pseudomonas aeruginosa strains are repeatedly carried out generally until the strains show the desired LD₅₀ values. Although the number of purifying procedures or steps varies depending on the level of skill of the technicians, the toxicity of parent strains and the like, the purification step is preferably carried out 3 to 7 times, e.g. until the LD₅₀ of the Pseudomonas aeruginosa is at least 2 x 10⁷ cells. The LD₅₀ level of novel attenuated Pseudomonas aeruginosa strains thus obtained according to the present invention in mouse is preferably 2.0x10⁷ cells or more.

The present invention also provides a vaccine for immunization against Pseudomonas aeruginosa infection and which is prepared by separating cell wall proteins in a pure state from each of the novel attenuated Pseudomonas aeruginosa strains according to the present invention as obtained above and then further purifying the separated cell wall proteins, and combining the purified cell wall proteins thus obtained in a certain mixing ratio, preferably in equivalent amounts of the cell wall proteins from each of the desired Pseudomonas aeruginosa strain for which immunization is sought. That is, the amount used for each strain is that sufficient to induce immunization against that strain in a particular host type.

In addition, the present invention provides a method for preparation of vaccines for immunization against Pseudomonas aeruginosa, characterized in that attenuated Pseudomonas aeruginosa strains are incubated in fermenters while maintaining optimum growth conditions to obtain a Pseudomonas aeruginosa strain mass. The obtained microorganism mass is treated according to a series of procedures including extraction of cell wall proteins by treatment with organic solvent, fractionation based on molecular weights, ultracentrifuging, to obtain the desired cell wall proteins. The obtained cell wall proteins derived from the seven (7) types of attenuated Pseudomonas aeruginosa strains are combined with each other in a suitable constitution at a desired ratio, preferably in equivalent amounts.

The method for the preparation of vaccines according to the present invention as described above can be summarized as follows.

**Table 1**

| Procedures for separation and purification of cell wall proteins from attenuated Pseudomonas aeruginosa strains and for preparation of vaccines |
|---|
| Cultivation of seven (7) types of attenuated Pseudomonas aeruginosa strains |
| ↓ |
| Separation of microorganism cells |
| ↓ |
| Separated cells + Organic Solvent |
| ↓ |
| Extraction of cell wall proteins |
| ↓ |
| First fractionation by molecular weight (Ultrafiltration) |
| ↓ |
| Second fractionation by molecular weight (Ultrafiltration) |
| ↓ |
| Ultracentrifuge(180,000g) |
| ↓ |
| Separated cell wall proteins (M.W. 10,000<<100,000) |
| ↓ |
| Pseudomonas aeruginosa vaccine |

Hereinafter, the vaccine compositions of the present invention will be more specifically explained with reference to the method for preparation thereof.

For preparing vaccines for prophylaxis against Pseudomonas aeruginosa according to the present invention, in the first step seven types of attenuated Pseudomonas aeruginosa strains, i.e. CFCPA 10142, CFCPA 20215, CFCPA 30720, CFCPA 40057, CFCPA 50243, CFCPA 60534 and CFCPA 70018 are respectively incubated in a suitable-sized fermenter. As the culture medium for this purpose, tryptic soy broth or,} preferably a special medium as described below, is suitable to the incubation of Pseudomonas aeruginosa as described above.

The special medium includes glucose (30g/l), peptone (15g/l), MgSO₄ (0.5g/l), CaCO₃ (5g/l), KH₂PO₄ (1g/l), FeSO₄ (5mg/l), CuSO₄ (5mg/l) and ZnSO₄ (5mg/l). This special medium is uniquely designed by the present inventors for the cultivation of Pseudomonas aeruginosa strains and is novel. Accordingly, this special medium is encompassed within the scope of the present invention. When Pseudomonas aeruginosa strains are grown in the special medium, the yield of the bacterial mass is at least 30% higher than the bacterial mass grown in tryptic soy broth on the basis of the weight of dry bacterial mass. Therefore, the use of such special medium is a preferred embodiment.

In such bacterial mass incubation step, the optimum culture conditions are: temperature 37°C, aeration rate 1 vvm (volume/volume. minute) and seed volume 5% v/v of the culture medium solution, and the incubation is carried out under the stirring rate of 100rpm for initial 2 hours and then 600 rpm for 10 to 20 hours, preferably for 12 to 16 hours. Upon the completion of incubation, in the second step

the precipitated bacterial cells are separated from the culture solution by means of centrifugation or the like. The separated bacterial cells are treated in the third step with an organic solvent to inactivate the microorganisms and at the same time to remove cell wall lipid components. In the third step, the organic solvent is preferably selected from the group consisting of acetone, chloroform, ethanol, butanol, etc., with acetone being most preferable.

Subsequently, the fourth step comprises the repeated extraction of cell wall proteins, i.e. 5 to 6 extractions, while not destroying the cells themselves such that their cell contents are lost. For this purpose, microorganism cells are retained in a solution such as phosphate buffer solution, tris buffer solution and the like, preferably in the phosphate buffer solution, and are acted on with a mixer or homogenizer, to extract the cell wall proteins. The extraction is preferably carried out by stirring at 100 to 2000 rpm at 4°C. In addition, in the fourth step special precautions should be taken to prevent the destruction of the cells so that the desired cell wall proteins can be kept separate from the toxic proteins present in cytoplasm. Thus, during the extraction procedure it is necessary to continuously determine whether the cells are being destroyed or not by determining the presence of lactate dehydrogenase or hexokinase as a cytoplasmic marker enzyme.

Thereafter, the cell wall proteins of the attenuated Pseudomonas aeruginosa strains obtained in a supernatant according to above procedures are fractionized by subjecting them to the first and second ultrafiltrations to obtain only the proteins having molecular weights between 10,000 and 100,000. In this step, it is very important that a molecular weight of the resulting cell wall proteins be within the range of 10,000 to 100,000. The reason is that the molecular weight of cell wall proteins less than 10,000 does not provide the desired prophylactic effect as determined by animal experiments, while a molecular weight higher than 100,000 may cause toxic effects due to the presence of high molecular lipopolysaccharide (LPS).

The separated cell wall proteins from each of the seven types of attenuated Pseudomonas aeruginosa having molecular weight between 10,000 and 100,000 are each further purified by ultracentrifugation to remove any possible minor lipopolysaccharide that may be present. Then a step to remove any bacterial substances is performed to finally obtain the cell wall proteins of attenuated Pseudomonas aeruginosa strains which are nonpathogenic and which have sufficient purity for use as a vaccine for prophylaxis against the Pseudomonas aeruginosa infection.

The cell wall proteins obtained from the seven types of attenuated Pseudomonas aeruginosa strains are then combined at a certain ratio to formulate the vaccine. When considering the frequency of occurrence of disease of the parent Pseudomonas aeruginosa strains prior to attenuation, the combination of cell wall proteins should be made by mixing cell wall proteins obtained from at least three (3) types, more preferably four(4) types or more, and most preferably all of the seven (7) types of the Pseudomonas aeruginosa strains. Although the mixing ratio varies with the types of Pseudomonas aeruginosa strains from which the cell wall proteins to be combined are obtained, the cell wall proteins are preferably mixed in a ratio of equivalent amounts.

For instance, the preferred vaccine compositions according to the present invention are those containing cell wall proteins obtained from CFCPA 10142, CFCPA 20215, CFCPA 30720 and CFCPA 60534 in a mixing ratio of 1:1:1.5:0.5; 1:1:1:1 or 0.5:1.5:1.5:0.5 or a vaccine containing all cell wall proteins obtained from CFCPA 10142, CFCPA 20215, CFCPA 30720, CFCPA 40057, CFCPA 50243, CFCPA 60534 and CFCPA 70018 in a mixing ratio of substantially equivalent amounts. The least amount of cell wall protein mixture from each of the strains of Pseudomonas aeruginosa present in the vaccine is that minimum amount which is sufficient to induce immunization in the intended host/patient.

If desired, the vaccine for prophylaxis against Pseudomonas aeruginosa infection according to the present invention can include, in addition to the cell wall protein components obtained as above, pharmaceutically acceptable excipients, for example, calcium hydroxide, calcium phosphate, ISCOM (immunostimulating complex), SAF-1 (Syntex Adjuvant Formulation-1), SAFm (modified Syntex Adjuvant Formulation) and similar excipients known to those skilled in the art.

For use as a prophylactic vaccine against Pseudomonas aeruginosa infection, the dosage to be administered varies with sex, age, weight, health condition, etc., of the subjects who may be exposed to Pseudomonas aeruginosa, but is generally 0.5 to 2.5mg of the mixture of cell wall protein from each of the attenuated strains. This amount is generally obtained from a bacterial mass of dry weight 0.1g to 0.5g (corresponding to wet weight 0.5 to 2.5g). The preferred route of administration is by intramuscular injection.

According to another aspect of the present invention, the cell wall protein obtained in a pure state from the attenuated Pseudomonas aeruginosa strain of the present invention can also be used as an antibody inducer to induce immunoglobulin in experimental animals. Therefore, the present invention provides a therapeutic agent for treating a Pseudomonas aeruginosa infection, which contains an immunoglobulin produced by injection of the cell wall protein in a pure state obtained by separation and purification as mentioned above, into an experimental animal to induce production of an immunoglobulin against Pseudomonas aeruginosa.

Specifically, the separated and purified cell wall protein obtained according to the procedures as specifically illustrated above, from attenuated Pseudomonas aeruginosa strains of the present invention is an antigen used to inoculate experimental animals, such as sheep, rabbit, etc., to induce the formation of the related antibody. The blood is collected from the experimental animals and then the serum is separated. The separated serum is treated in a known manner to obtain the desired immunoglobulin in a purified state. For this purpose, the separation and purification of immunoglobulin from the separated serum can be practiced according to the method well-known in the relevant technical field (see Practical Immunology, 3rd Ed. (1989), 292-294], for example, by mixing the separated serum with distilled water, adding the mixture to DEAE-cellulose (diethylaminoethyl-cellulose) to allow the immunoglobulin to be absorbed, removing the supernatant and then washing immunoglobulin-absorbed DEAE-cellulose several times with phosphate buffer solution to obtain the purified immunoglobulin.

The immunoglobulin used in the therapeutic agent for treating Pseudomonas aeruginosa infection is obtained by immunizing an experimental animal with a mixture containing cell wall proteins separated from various attenuated Pseudomonas aeruginosa strains in a certain ratio. For this purpose, a combined immunoglobulin obtained by immunizing the experimental animal with a mixture consisting of each of cell wall proteins obtained from each of the seven (7) types of attenuated Pseudomonas aeruginosa strains in a certain ratio, preferably in a ratio of equivalent amounts can be used. However, when the cross reactivity of each of the immunoglobulins is considered, the immunoglobulins obtained by immunizing an experimental animal with a mixture consisting of cell wall proteins separated from each of the 4 types of attenuated Pseudomonas aeruginosa strains as follows: CFCPA 10142, CFCPA 20215, CFCPA 30720 and CFCPA 60534 and in a ratio of 1:1:1:1 provides, considerable therapeutic effect on infections caused by all of the Pseudomonas aeruginosa strains having Fisher types 1, 2, 3, 4, 5, 6 and 7. This is the preferred combined immunoglobulin therapeutic agent for treating a desease caused by Pseudomonas aeruginosa.

The immunoglobulin for Pseudomonas aeruginosa according to the present invention can be formulated into pharmaceutical compositions in a lyophilized form or in a liquid form and, if necessary, additionally contain pharmaceutically acceptable carriers, for example, stabilizers, preservatives, isotonic agents, and the like. The pharmaceutically acceptable carriers which can be used preferably include, for example, mannitol, lactose, saccharose, human albumin, etc. in the case of lyophilized preparations and physiological saline, water for injection, phosphate buffer solution, aluminum hydroxide, etc. in the case of liquid preparations.

The dosage of immunoglobulin varies depending on the age, body weight, sex and general state of health of the subject, the severity of Pseudomonas aeruginosa infection and the components of the combined immunoglobulin being administered. The immunoglobulin is generally administered in an amount of 0.1mg to 1000mg, preferably 1mg to 100mg, per day per kg body weight to an adult via the intravenous route.

The present invention will be more specifically illustrated by the following examples but is not limited in any way to the examples.

### Example 1 : Isolation of Pseudomonas aeruginosa strain from Pseudomonas aeruginosa infected patients and identification thereof

From each of 260 different samples of blood taken from Pseudomonas aeruginosa infected patients, about 1 to 5 ml ali- quot was aseptically collected and allowed to stand for one hour at room temperature. After the blood was stored overnight in a refrigerator at 4°C, it was centrifuged with 1500xg (g:gravity) for 10 minutes at 4°C to remove the solid substances and to obtain a supernatant serum. The serum obtained above was diluted with phosphate buffer solution (NaH₂PO₄ 1.15g, KCl 0.2g. KH₂PO₄ 0.2g, NaCl 8.766g per liter) in a ratio of 1:10 (serum to solution) and then spread on a tryptic soy broth culture plate adding 1.0 to 1.5% agar, which was previously prepared. The culture plate was incubated for 12 hours, or more, in an incubator maintained at a constant temperature of 37°C under aerobic conditions. The cultures thus obtained were transferred to fresh culture plates and then the desired microorganisms were isolated in a pure state by means of a known microorganism pure isolation method [see, Thomas D. Brock and Michael T. Madigan, Biology of Microorganisms (1988), 5th Ed. pp32-33, Prentice Hall, Englewood Cliffs, New Jersey].

For the isolated Pseudomonas aeruginosa strains, their serological and immunological characterizations have already been disclosed (see, Bergey's Manual) and their identification can be determined using a commercial analysis kit according to the analytical method described in J. Gen. Microbiol, 130, 631-644, 1984. Each Pseudomonas aeruginosa strain was inoculated into a test tube containing 5ml of tryptic soy broth and then incubated at 37°C under aerobic conditions to adjust the concentration of bacterial mass so as to be maintained the absorbance of approximately 2.0 or more under visible light of 650nm.

An aliquot of 1ml was aseptically taken from this culture solution and centrifuged with 6000xg at 4°C for 10 minutes. The supernatant culture solution was removed and the precipitated microorganism was suspended by adding the same amount of sterilized saline. 15 ul of each of test serum contained in the kit and control serum (normal rabbit serum) were added to each well of a 96-well microplate and were mixed with 15 ul of microorganism suspension obtained above to determine whether the agglutination occurs within 10 to 30 minutes. In this study, since the microorganism strain in a well with positive agglutination has a serotype identical with that of serum added thereto, the identification of the isolated microorganisms could be readily achieved.

### Example 2 : Selection of attenuated Pseudomonas aeruginosa strains from each isolated Pseudomonas aeruginosa strains

In order to attenuate Pseudomonas aeruginosa strains to obtain safe microorganisms for use in the preparation of a vaccine, one strain for each of the Pseudomonas aeruginosa strains having different immunotypes was selected and then attenuated through repeated purifications.

In this procedure, the control for a toxic Pseudomonas aeruginosa strain GN 11189 (Episome Institute, Japan) was selected, which shows a lethality rate of 100% within 3 days after intravenous (or intraperitoneal) injection of 2.0x10⁶ cells of GN 11189 into a male ICR mouse weighing 20 to 25g.

Each strain of the above selected Pseudomonas aeruginosa strains having a different immunotype was cultured in liquid tryptic soy broth under the same conditions as in Example 1 and then centrifuged with 6000xg at 4°C for 10 minutes. The obtained cell precipitate was suspended in 10ml of physiological saline, centrifuged again under the same conditions as above, and then diluted with fresh physiological saline to adjust the cell content to 5x10⁵, 5x10⁶, 5x10⁷ and 5x10⁸ cells per ml. Each cell dilution was administered to one group consisting of 10 ICR mice via the intravenous (or intraperitoneal) route. To the control group, the GN 11189 strain was administered in an amount of 2.0x10⁶ cells per control animal. At the point where the lethality within 3 days in the control group is 100%, Pseudomonas aeruginosa strains were aseptically isolated from ICR mice/mouse survived at the highest cell concentration. The isolated Pseudomonas aeruginosa strains were spread again on tryptic soy agar plate medium. Following microorganism pure isolation method as mentioned above each microorganism strain was isolated in a pure state.

All seven (7) kinds of the first attenuated microorganisms were repeatedly passed through ICR mice about 3 to 7 times according to the method described above until the desired LD₅₀ of at least 2.0x10⁷ cells was achieved for each microorganism strain. The safety value of each finally attenuated Pseudomonas aeruginosa strain, which is expressed as LD₅₀ is listed on the following Table 2.

**Table 2**

| Safety values of attenuated Pseudomonas aeruginosa strains selected according to the present invention | | | | |
|---|---|---|---|---|
| | Selected P. aeruginosa strains | Fisher-Devlin Immunotype | Initial LD₅₀ | Final LD₅₀ |
| Test Strain | CFCPA 10142 | 1 | < 1.5x10⁶ | 7.6x10⁷ |
| | CFCPA 20215 | 2 | < 1.4x10⁶ | 2.7x10⁷ |
| | CFCPA 30720 | 3 | < 2.0x10⁶ | 2.5x10⁸ |
| | CFCPA 40057 | 4 | < 3.7x10⁶ | 4.5x10⁷ |
| | CFCPA 50243 | 5 | < 5.0x10⁶ | 3.0x10⁷ |
| | CFCPA 60534 | 6 | < 1.5x10⁶ | 2.0x10⁷ |
| | CFCPA 70018 | 7 | < 3.8x10⁶ | 2.7x10⁷ |
| Control Strain | GN 11189 | - | - | 2.0x10⁶ |

All of seven (7) kinds of microorganisms as mentioned above exhibited the LD₅₀ value of at least 2.0x10⁷ cells. Accordingly, it could be identified that the attenuated microorganisms were selected.

### Example 3 : Identification of attenuated Pseudomonas aeruginosa strain

Sterilized cetrimide 10% (w/v) was added to a liquid nutrient medium (pH 7.2-7.4), which was prepared by steam sterilization in an autoclave at 121°C for 15 minutes, to a final concentration of 0.3% (v/v). A 10ml aliquot of the medium thus obtained was aseptically taken and then introduced into a test tube, to which one drop of each attenuated Pseudomonas aeruginosa strain isolated in a pure state according to Example 2, above, was inoculated and then subjected to seed culture at 30 to 37°C for 12 to 16 hours. From the test tube in which the growth of microorganism occurs, 0.5ml of the culture solution was taken, spread on a plate of cetrimide agar medium as Pseudomonas aeruginosa isolation medium and then the plate was incubated. The colony first identified as Pseudomonas aeruginosa by pigment formation was transferred and incubated on a Pseudomonas aeruginosa agar medium for detection of fluorescin (Proteose peptone No. 3 (oxoid) 2%, Glycerol (bidistilled) 1%, K₂HPO₄ (anhydrous) 0.15%, MgSO₄.7H₂O 0.15%, Agar 1.5%(w/v), pH7.2) and then a Pseudomonas aeruginosa agar medium for detection of pyocyanin (Peptone (Difco) 2%, Glycerol (bidistilled) 1%, K₂SO₄ (anhydrous) 1%, MgCl₂ (anhydrous) 0.14%, Agar 1.5% (w/v), pH7.2) and then examined again for morphological test, nutritional requirement and oxidase test to determine the presence of the Pseudomonas aeruginosa strain. The types of the selected Pseudomonas aeruginosa strains were determined according to the IATS classification system by using a Pseudomonas aeruginosa antigen kit (produced by Difco Laboratories, Detroit, Michigan, USA) and then classified by Fisher immuno-serotypes. The results thereof are described in the following Table 3.

**Table 3**

| Criteria for selection of Pseudomonas aeruginosa strains and their serotype and protective range | |
|---|---|
| Test item | Test results |
| Growth on isolation medium | Growth |
| Growth on Pseudomonas aeruginosa medium for detection | Growth |
| Morphological features (Microscope) | Both ends are round and a single polar flagellum is present. |
| Gram staining | Gram negative |
| Oxidase reaction | Positive response(+) or positive-negative response (+) |

| Antigen-Antibody reaction (first selected strains) | |
|---|---|
| CFCPA 20215 | Protective for Fisher types 3 and 7 as Fisher immunotype 2 (O-serotype 7) |
| CFCPA 60534 | Protective for Fisher types 3 and 7 as Fisher immunotype 6 (O-serotype 5) |
| CFCPA 10142 | Protective for Fisher types 2 and 1 as Fisher immunotype 1 (O-serotype 4) |
| CFCPA 30720, CFCPA 30721 | Protective for Fisher type 6 as Fisher immunotype 3 (O-serotype 3) |

### Example 4 : Physiological characteristics of attenuated Pseudomonas aeruginosa strains

(1) Each of the seven kinds of Pseudomonas aeruginosa strains attenuated according to Example 2, growth rates depending on different pH values were determined. Each microorganism strain was inoculated into 50ml of tryptic soy broth with a pH value of 7.2, and then pre-incubated at 37°C for 12 to 16 hours under aerobic conditions with the agitation rate of 200 rpm. Each pre-incubated bacterial mass strain was inoculated into 500ml of each of fresh tryptic soy broth of pH 3.0, pH 5.0, pH 7.0 and pH 9.0 in the final concentration of 1% (v/v) and then incubated in fermenters at 37°C under the same conditions as above. During this period, a sample was obtained from each culture medium at an interval of 2 hours and then the concentration of bacterial mass was determined by measuring the absorbance of the sample at 600nm by means of spectrophotometer. The results thereof are described in the following Table 4.

**Table 4**

| Microorganism growth rates depending on pH value | | | | |
|---|---|---|---|---|
| Strains | pH 3.0 | pH 5.0 | pH 7.0 | pH 9.0 |
| CFCPA 10142 | - | +++ | +++ | +++ |
| CFCPA 20215 | - | +++ | +++ | +++ |
| CFCPA 30720 | - | +++ | +++ | ++ |
| CFCPA 40057 | - | +++ | +++ | ++ |
| CFCPA 50243 | - | +++ | +++ | ++ |
| CFCPA 60534 | - | +++ | +++ | ++ |
| CFCPA 70018 | - | +++ | +++ | +++ |
| Note : + Growth, - No growth | | | | |

As can be seen from Table 4, the attenuated Pseudomonas aeruginosa strains according to the present invention did not grow under excessively acidic conditions, for example, at pH 3.0, but showed substantially identical or similar growth rates under medium conditions at pH 5.0 to pH 9.0. Therefore, in the present invention all 7 kinds of microorganisms can grow very well within a wide pH range.
(2) According to the same method as in (1), above, the growth rates of Pseudomonas aeruginosa strains were determined according to temperature variations. Each of the 7 kinds of attenuated Pseudomonas aeruginosa strains was inoculated into 50ml of tryptic soy broth at a pH value of 7.0 and then preincubated for 12 to 16 hours at 37°C under aerobic conditions with the agitation rate of 200rpm. Each of the pre-incubated strains was inoculated into 500ml of tryptic soy broth at a pH value of 7.0 and then incubated in fermenters, which were maintained at different temperatures of 25°C, 30°C, 37°C and 42°C, under the same conditions as above. During this period, the growth rate in each fermenter was determined by measuring the absorbance of the culture solution at 600nm by means of spectrophotometer. The results are described in Table 5.

**Table 5**

| Microorganism growth rates depending on temperature variations | | | | |
|---|---|---|---|---|
| Strains | 25°C | 30°C | 37°C | 42°C |
| CFCPA 10142 | + | +++ | +++ | + |
| CFCPA 20215 | + | +++ | +++ | + |
| CFCPA 30720 | ++ | +++ | +++ | + |
| CFCPA 40057 | + | ++ | +++ | + |
| CFCPA 50243 | + | ++ | +++ | + |
| CFCPA 60534 | ++ | +++ | +++ | + |
| CFCPA 70018 | + | ++ | +++ | + |
| Note : + Growth, - No growth | | | | |

As can be seen from Table 5, all 7 kinds of attenuated Pseudomonas aeruginosa strains showed the best growth at 37°C and also showed the good growth at 30°C, 25°C. However, at 42°C each of the seven kinds of Pseudomonas aeruginosa strains showed a relatively slow growth rate relative to the growth rate at the other temperatures.
(3) The following experiment was to determine the effect of carbon source on the growth rate of attenuated Pseudomonas aeruginosa strains. Each of the 7 kinds of attenuated Pseudomonas aeruginosa strains was inoculated into 50ml of tryptic soy broth at a pH value of 7.0 and then incubated at 37°C for 12 to 16 hours under aerobic conditions with the agitation rate of 200rpm. The culture medium was then centrifuged with 6000xg at 4°C for 10 minutes under aseptic conditions to harvest the bacterial mass which was then resuspended in 10ml of physiological saline. Each 50 ul of the microorganism suspensions obtained above was, respectively, inoculated to 5ml of M9 medium (Na₂HPO₄ 7H₂O 12.8g, KH₂PO₄ 3g, NaCl 0.5g, NH₄Cl 1g) containing different carbon sources (as see below) and then incubated for 12 hours. Then, for each culture solution the extent of microorganism growth was determined by measuring the absorbance at 600nm.

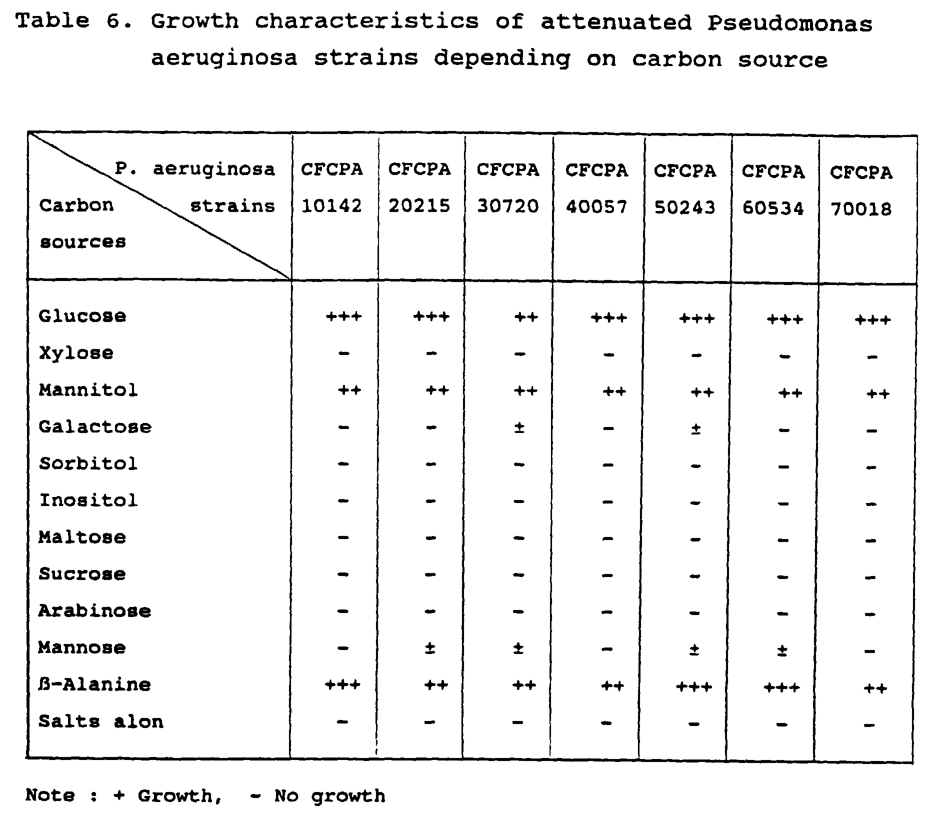

Growth characteristics of attenuated Pseudomonas aeruginosa strains depending on carbon sources exhibited a pattern substantially similar to the growth characteristics of general Pseudomonas aeruginosa strains depending on carbon sources (see Bergey's Manual). However, it should be noted that even though Pseudomonas aeruginosa has been reported as generally not using mannose, attenuated Pseudomonas aeruginosa according to the present invention exhibited some growth in the mannose-containing medium.

As can be seen from the above, attenuated Pseudomonas aeruginosa strains according to the present invention can be incubated in a conventional medium containing a carbon source, nitrogen source, inorganic compounds, amino acids, vitamins and other nutrients under aerobic conditions at selected temperature, pH, etc., to obtain a bacterial mass. Cell wall proteins from the resulting bacterial mass are used for preparation of Pseudomonas aeruginosa vaccines.

As the carbon source for incubation of attenuated Pseudomonas aeruginosa strains, various carbohydrates such as glucose, mannitol, fructose, etc., various organic acids such as acetic acid, pyruvic acid, lactic acid, and the like, can be used. For the nitrogen source various organic and inorganic ammonium salts, peptone, yeast extracts, casein hydrolysates and the other nitrogen-containing substances can be used. As inorganic compounds magnesium sulfate, calcium carbonate, ferrous sulfate, cuprous sulfate, zinc sulfate and potassium hydrogen phosphate and potassium dihydrogen phosphate and the like can be used. The incubation is preferably carried out at 25 to 37oC under aerobic conditions, for example, by means of a plate culture or of a liquid culture such as shaking culture or aeration spinner culture. The pH value of medium is preferably maintained at pH 5 to 9 during the period of incubation. Preferably, the bacterial mass obtained by centrifuge of the culture solution incubated for 12 to 16 hours is used as starting material for preparation of vaccines as described hereinafter.

### Example 5 : Incubation of attenuated Pseudomonas aeruginosa strains

(1) In a 5L fermenter the bacterial mass of Pseudomonas aeruginosa obtained according to Example 2 was incubated for mass production, as follows. 2.5L of the medium solution obtained by dissolving 30g of tryptic soy broth per 1L of distilled water was adjusted to pH 7.2, sterilized and then introduced into the 5L fermenter. The incubation conditions were: incubation temperature was 37°C; aeration rate was 1 vvm ; inoculation amount was 5% (v/v) of attenuated Pseudomonas aeruginosa bacterial mass obtained from Example 2 for culture solution; and the agitation rate was maintained at 100 rpm for the initial 2 hours and, thereafter, the incubation was continued for 12 to 16 hours at the fixed agitation rate of 600rpm. Although the amounts of obtained bacterial mass were somewhat different depending on the type of the Pseudomonas aeruginosa strain, the average yield was approximately 8g (dry weight) of bacterial mass per liter of culture solution.
(2) Each of the seven types of attenuated Pseudomonas aeruginosa strains was incubated under the same incubation conditions as in the above (1), except that the special medium for culturing Pseudomonas aeruginosa which consists of glucose (30g/l), peptone (15g/l), MgSO₄ (0.5g/l), CaCO₃ (5g/l), KH₂PO₄ (1g/l), FeSO₄ (5mg/l), CuSO₄ (5mg/l) and ZnSO₄ (5mg/l), is used as a medium. With this special medium approximately 10.4g to 10.5g (dry weight) of bacterial mass for each microorganism strain was obtained. Thus, the use of this special medium can provide the yield of bacterial mass which is at least 30% (based on dry weight) higher than that in tryptic soy broth.

### Example 6 : Partial purification of cell wall proteins from attenuated Pseudomonas aeruginosa strains

For preparing vaccines against Pseudomonas aeruginosa, cell wall proteins from each of the seven kinds of attenuated Pseudomonas aeruginosa strains were partially purified. Hereinafter, the type 3 strain, i.e. CFCPA 30720(KCCM 10031), is used as a typical example. However, the other attenuated Pseudomonas aeruginosa strains can also be subjected to the same method as the following purification method to obtain the partially purified cell wall proteins.

2.5L of the special medium as used in Example 4(2) above was introduced into a 5L fermenter and then incubated for 12 to 16 hours while maintaining the same incubation conditions as above. Upon the completion of incubation, 2.5L of the culture solution was centrifuged with 6000xg at 4°C for 20 minutes to remove the supernatant. The precipitated cells were resuspended in a phosphate buffer solution (Na₂HPO₄ 1.15g, KCl 0.2g, KH₂PO₄ 0.2g, NaCl 8.766g, per liter, pH 7.2) at 4°C, centrifuged again and then washed with the same phosphate buffer solution. To 130g of the cells thus obtained was added 3 volumes (about 390ml) of acetone with respect to the original wet cell volume and the mixture was allowed to stand at 4°C for 12 hours or more. The acetone was removed from the precipitated cell and 2 volumes (about 260ml) of fresh acetone with respect to the original wet cell volume was added again to the residue. The resulting mixture was stirred for 5 to 10 minutes and then centrifuged with 8000xg at 4°C for 20 minutes. The supernatant acetone was removed and to the residue the same volume of acetone was added. The mixture was stirred and centrifuged according to the same manner described above to remove the supernatant. The precipitated cells were dried on a plate at room temperature to remove acetone. The dried microorganisms were resuspended by adding 250ml of the same phosphate buffer solution as above so that the final concentration can be adjusted to 10% (v/v). The resulting mixture was treated with a homogenizer at the rate of 500 to 1500 rpm for 10 minutes while maintaining the temperature of 4°C to extract cell wall proteins. The obtained suspension was centrifuged with 8000 to 10000xg for 30 minutes to obtain the supernatant. The precipitated cells were separated, suspended again by adding the same volume of phosphate buffer solution and then subjected to a second extraction under the same conditions as the above first extraction to obtain the supernatant.

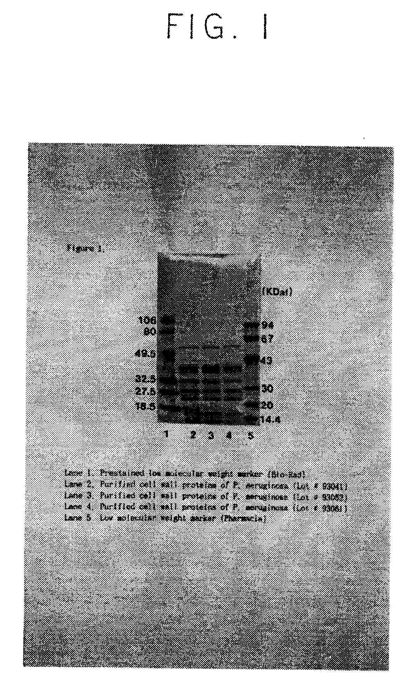

By using the same conditions as the first extraction method, the cell wall proteins were repeatedly extracted 5 to 6 times while taking care to not destroy (lyse) any of the cells. The destruction of the cells can be determined by measuring a specific protein as a cytoplasmic marker substance, i.e. lactate dehydrogenase or hexokinase. The extracted supernatants, each of which is tested to ensure that the cell wall proteins are extracted without the incorporation of cytoplasmic proteins, lactate dehydrogenase and hexokinase, were collected, stirred and then finally recentrifuged with 10000xg at 4°C for 30 minutes to obtain a clear supernatant. The protein solution thus obtained was composed only of substantially pure cell wall proteins and was adjusted with protein quantitative assay so that the protein concentration is maintained at the level of 1mg/ml to 2mg/ml. Further, the purity of the protein solution was determined by means of 10 to 15% concentration gradient electrophoresis. As a result, it could be identified that presence of the desired cell wall proteins having the molecular weight ranging from 10,000 to 100,000 were present (see Figure 1).

### Example 7 : Pure purification of crude proteins

The following process was carried out so that the crude cell wall protein solution in the concentration of 1 to 2 mg/ml would be purified in order to contain only the effective components.

2 to 2.5L of crude cell wall protein solution was first subjected to a molecular weight 100,000 cut-off membrane filter in the Pellicon Cassette System to remove protein molecules having molecular weight over 100,000. According to this process, proteins having molecular weight below 100,000 were recovered as filtrate and proteins having molecular weight greater than 100,000 were continuously circulated to be separated from the small molecules having molecular weight below 100,000.

As a result the protein solution was concentrated by 10 to 20% of the initial volume and was successively washed with 20 to 25L (ten times amount of the initial volume of protein solution) of sterilized phosphate buffer solution (Na₂HPO₄ 1.15g, KCl 0.2g, KH₂PO₄ 0.2g, NaCl 8.766g, per liter) to recover 90% or more of proteins having molecular weight below 100,000. The proteins having molecular weight below 100,000 separated as the filtrate were applied to a molecular weight 10,000 cut-off membrane filter in the same system to remove proteins having molecular weight less than 10,000 and other impurities and at the same time to concentrate the proteins having molecular weight of 10,000 to 100,000 to a concentration of 1mg/ml.

Finally, the supernatant was ultracentrifuged to remove lipopolysaccharide (LPS) and cell wall-related fragments, which are possibly present in the supernatant obtained above in trace amounts. Ultracentrifuging is carried out with 180,000xg to 200,000xg for 3 hours at 4°C. After removing the precipitates, the obtained supernatant was sterilized by filtration through 0.2um filter to obtain 250 to 260mg of protein composition for use in vaccines for prophylaxis against Pseudomonas aeruginosa infection.

### Example 8 : Purification of cell wall proteins from attenuated Pseudomonas aeruginosa strains having different immunotypes

The remaining six kinds of attenuated Pseudomonas aeruginosa strains according to the present invention, i.e. CFCPA 10142, CFCPA 20215, CFCPA 40057, CFCPA 50243, CFCPA 60534 and CFCPA 70018 were treated according to the same method as that for microorganism incubation and purification to prepare vaccine composition for CFCPA 30720 Pseudomonas aeruginosa strain as described in Example 5, Example 6 and Example 7. The final Pseudomonas aeruginosa vaccine compositions thus obtained showed the same band pattern as CFCPA 30720 vaccine composition, in 10 to 15% concentration gradient SDS-PAGE. In addition, as a result of comparison with standard molecular weight marker it could be determined that all proteins contained in vaccine compositions have the molecular weight ranging from 10,000 to 100,000 (Figure 1).

### Example 9 : Cross-protective capacity test with combined antigens

The cell wall proteins derived from each attenuated Pseudomonas aeruginosa strain after the separation and purification steps according to the methods described in Examples 7 and 8 were intraperitoneally administered in an amount of 0.2mg/kg to 6 weeks-old ICR male mouse weighing 23 to 25g to immunize the animal. Each group consisted of 10 to 15 test animals. One week from the immunization, blood was taken from 2 to 3 mice per group for ELISA (Enzyme-linked immunosorbent assay). For the remaining ICR mice wild-type Pseudomonas aeruginosa strain corresponding to the respective immunotype was injected in an amount of 10 to 50 times the initial LD₅₀ value for each microorganism strain listed on Table 2 in Example 2. The protective efficacy against each Pseudomonas aeruginosa strain was continuously examined for one week. As a result, all the test groups showed a protective effect against the corresponding Pseudomonas aeruginosa strains and the ELISAs to all the collected blood also exhibited good positive values, which mean an improvement in the immunological responses.

In the present invention, for preparing vaccines capable of exhibiting the common protective effect against infections caused by any type of Pseudomonas aeruginosa strains, four types of attenuated Pseudomonas aeruginosa strains (three types of Pseudomonas aeruginosa strains which are most frequently detected in human body infections and one type of Pseudomonas aeruginosa strain which is difficult to overcome if contracted, i.e. CFCPA 10142, CFCPA 20215, CFCPA 30720 and CFCPA 60534) were treated to obtain cell wall proteins which were combined at a ratio of equivalent amounts and then were examined for cross protective capacity against each immunotype of Pseudomonas aeruginosa strains.

Cell wall proteins separated from the four types of attenuated Pseudomonas aeruginosa strains, as stated above, were combined at a ratio of equivalent amounts (1:1:1:1) based on weight. The combined cell wall proteins were intraperitoneally administered to ICR male mice to immunize the test animals. The control group was given 0.3ml of phosphate buffer solution (Na₂HPO₄ 1.15g, KCl 0.2g, KH₂PO₄ 0.2g, NaCl 8.766g, per liter). After one week from immunization, each of the test group immunized with Pseudomonas aeruginosa vaccine and the control group immunized with phosphate buffer solution was intraperitoneally given Pseudomonas aeruginosa in five (5) different concentrations diluted in a series of 10 times from 1x10⁸ cells to 1x10⁴ cells, and after on week, the survival number of test animals was counted to calculate the efficacy index (EI). The results thereof are listed in Table 7, below, in which the efficacy index is defined as a value of LD₅₀ in the test group divided by the LD₅₀ in the control group. As can be seen from Table 7, a vaccine consisting of cell wall proteins obtained from 4 kinds of attenuated Pseudomonas aeruginosa strains, i.e. CFCPA 10142, CFCPA 20215, CFCPA 30720 and CFCPA 60534 has an EI value of 3.0 to 18 or more against each immunotype of Pseudomonas aeruginosa strains. Therefore, when considering that vaccines having the EI value of at least 2 are regarded as having a good immunological effect, it can be seen that the vaccine according to the present invention shows a good protective effect against infections caused by any type of the 7 kinds of Pseudomonas aeruginosa strains.

**Table 7**

| Cross-protective capacity test for Pseudomonas aeruginosa vaccine prepared using four (4) different species of attenuated Pseudomonas aeruginosa strains against each type of microorganisms | | | |
|---|---|---|---|
| Challenge Strain | Immunotype | Serotype | EI value |
| Parent strain of CFCPA 10142 | 1 | 06 | 9.5 |
| Parent strain of CFCPA 20215 | 2 | 011 | 13 |
| Parent strain of CFCPA 30720 | 3 | 02 | >18 |
| Parent strain of CFCPA 40057 | 4 | 01 | 3 |
| Parent strain of CFCPA 50243 | 5 | 010 | 4.5 |
| Parent strain of CFCPA 60534 | 6 | 07 | 8.4 |
| Parent strain of CFCPA 70018 | 7 | 05 | 7 |

According to the procedure as mentioned above, except that the mixture of cell wall proteins prepared were derived from 3 types of attenuated Pseudomonas aeruginosa strains CFCPA 10142, CFCPA 30720 and CFCPA 20215 (Group I), and the mixture of cell wall proteins prepared from all 7 types of attenuated Pseudomonas aeruginosa strains (Group II), wherein all the cell wall proteins are present in a ratio of equivalent amount, are used instead of the mixture of 4 kinds of proteins, the cross-protective capacity of these 2 kinds of vaccines against infection of each Pseudomonas aeruginosa was examined. The results thereof are described in Table 8.

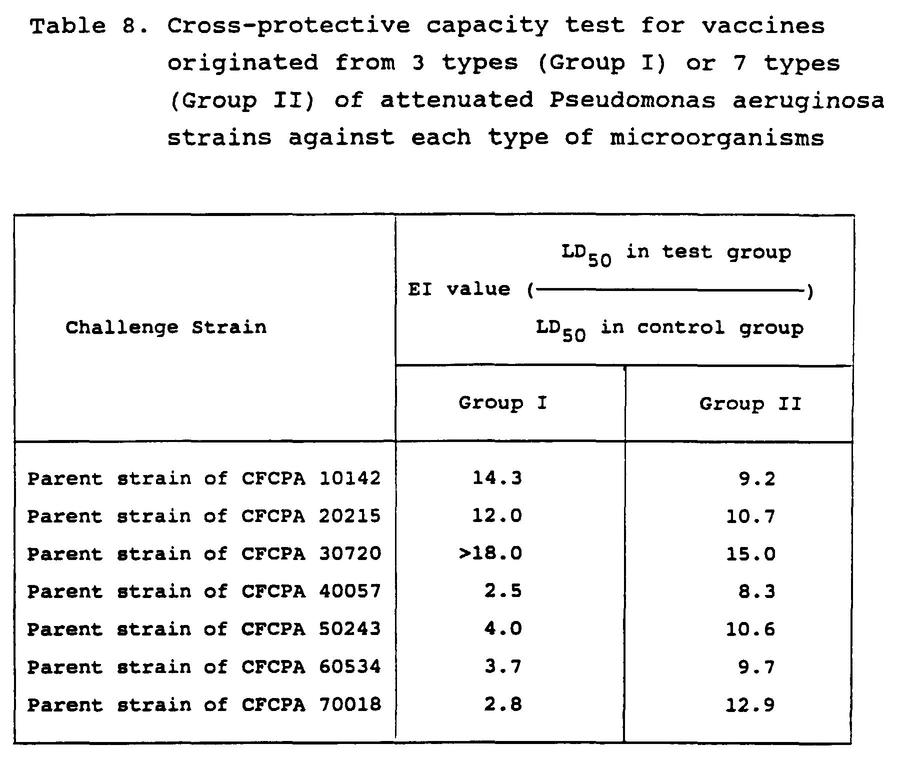

As can be seen from the above experimental results, since the mixed protein components vaccine according to the present invention is composed of cell wall proteins prepared from at least three (3) different kinds of attenuated Pseudomonas aeruginosa strains showing different immunotypes from each other, this vaccine shows excellent protective effect against any immunotype of Pseudomonas aeruginosa in comparison with only one kind of common antigen. That is, although the efficacy index of the vaccine according to the present invention varies with the kind and number of selected microorganisms from attenuated Pseudomonas aeruginosa strains, mixing ratio of cell wall proteins derived therefrom, immunological schedule and method, etc., it can be confirmed that the vaccine of the present invention is effective against all Pseudomonas aeruginosa strains presently occurring in hospitals and patients.

### Example 10 : Toxicological test for cell wall proteins

To prepare a component vaccine for prophylaxis against infection caused by Pseudomonas aeruginosa, the safety of cell wall proteins themselves to be used as component vaccine, as well as the safety of microorganism strains themselves as described in Example 2, should be assured. In this example, cell wall proteins were partially purified and then examined for their safety in an experimental animal, i.e. mouse. Specifically, each of attenuated microorganisms was incubated with tryptic soy broth as the culture medium, in a 5L fermenter and then treated according to the procedures described in Examples 5, 6 and 7. The supernatants of cell wall proteins extracted from attenuated Pseudomonas aeruginosa strains were collected together, centrifuged again with 10000xg at 4°C for 30 minutes to remove fine particles possibly present in solution. The resulting cell wall proteins were filtered through an Amicon membrane filter to obtain a mixture of cell wall proteins having molecular weight ranging from 10,000 to 100,000, which was concentrated to a protein concentration of 1mg/ml and then sterilized with a 0.2um filter to obtain a protein source for toxicological testing.

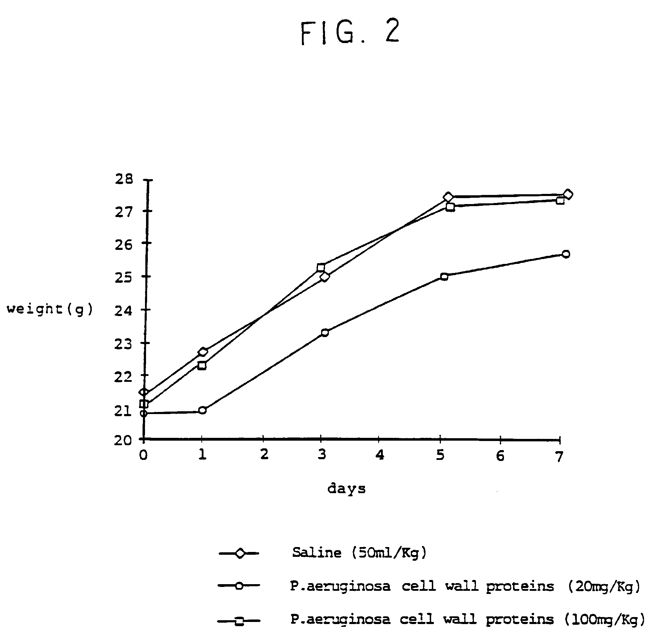

In the toxicological test, the experimental animals used were ICR male mice weighing from 20 to 22g. To the test group, the protein source was intravenously injected in an amount of 20mg/kg and 100mg/kg. The control group was given physiological saline in an amount of 25ml/kg. As can be seen from Table 9 and the attached Figure 2, the test group exhibited a slight inhibition to weight gain on the first day after administration but on and after the second day after administration showed a normal growth and no special symptoms. In addition, even on and after the 7th day no specific change or symptoms in each organ could not be found.

**Table 9**

| Acute toxicological test for cell wall protein source of attenuated Pseudomonas aeruginosa strains | | | | |
|---|---|---|---|---|
| Group | Dosage | Test Subjects | Survival/Test Subjects | Remarks |
| Test Group A | 20mg/kg | 20 | 20/20 | No change of body weight |
| Test Group B | 100mg/kg | 15 | 15/15 | Change of body weight only on the first day |
| Control Group | Physiological Saline | 15 | 15/15 | No change of body weight |

### Example 11 : Determination of antigenicity of cell wall proteins

Cell wall proteins obtained by partial purification of attenuated Pseudomonas aeruginosa strains in the same manner as in Example 10 were used as an antigen and were intravenously injected to ICR mice in an amount of 0.1mg/kg or 0.2mg/kg to immunize the experimental animals. One week from immunization, a certain amount of blood was taken from each mouse of the group of mice, the serum was separated from the collected blood and the formation of the antibody was determined in accordance with ELISA method [J. Clin. Microbiol. 15, 1054-1058, 1982].

First, 100 ul of antigen prepared in Example 10 which was adjusted to a concentration of 0.1mg/ml in a coating buffer (0.05M carbonate buffer solution consisting of NaHCO3 2.85g, Na₂CO3 1.70g, H₂O 1 , pH 9.6) was added to each well of a 96-well microplate and then reacted either at room temperature for 2 hours or at 4°C for 12 to 14 hours for the protein to adhere to the plate.

After removing the aqueous solution 200 ul of 1% bovine serum albumin (BSA) was added to the each plate well and then reacted at room temperature for one hour to block the remaining portion in the wells not bound to protein.

In this test, serum of a non-immunized mouse was used as an antibody for the control group. Upon completion of the reaction, the plate was washed again 5 to 6 times with a phosphate buffer solution (pH7.0-7.2) and 100 ul of a second antibody, i.e. Rabbit-anti mouse Ig-Peroxidase Conjugate, was added to each well and allowed to react at normal temperature for one hour.

Thereafter, the plate was vigorously washed 7 times or more with the same phosphate buffer washing solution (pH7.0-7.2). As a substrate 50 ul of ortho-phenylenediamine dihydrochloride, adjusted to a concentration of 0.3 to 0.4mg/ml in citrate-phosphate buffer solution (0.1M Citrate-Phosphate buffer, pH5.0), was added to each well of the plate and reacted for 20 minutes in the absence of light. Then 50 ul of 1N-sulfuric acid was respectively added to stop the reaction in each well. Then, for each reaction solution the absorbance at 490nm was measured by means of a spectrophotometer.

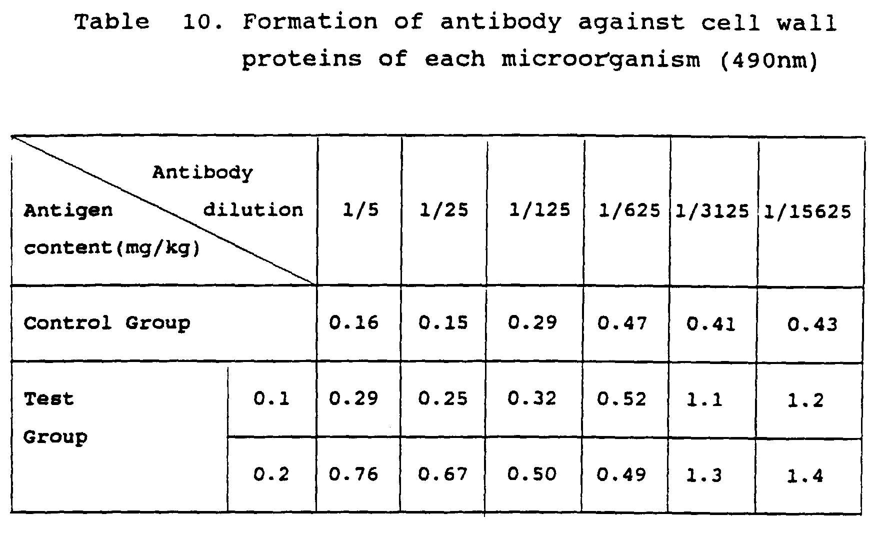

As can be seen from Table 10, in the test group the administration of 0.1 or 0.2mg/kg antigen results in the immunity 2 to 5 times higher than that in the control group.

### Example 12 : Production of immunoglobulin for Pseudomonas aeruginosa

The protein solution obtained from Example 7 was administered to rabbits to produce the corresponding immunoglobulin. Although the cell wall protein obtained from attenuated Pseudomonas aeruginosa strain CFCPA 30720 was used as a typical example, the same method can be applied to the other attenuated Pseudomonas aeruginosa strains.

0.5 to 1ml (containing 100 to 200 ug of cell wall protein) of the cell wall protein solution obtained from CFCPA 30720 strain in Example 7 was used to inoculate albino rabbits three times at intervals of 7 days. Then blood was taken at regular intervals from each of the rabbits and the serum was separated. 100ml of the separated rabbit serum was mixed with 300ml of distilled water and the mixture was added to 500g (wet weight) of DEAE-cellulose at 4°C The resulting mixture was thoroughly shaken for one hour at 4°C, allowed to stand and then the supernatant was separated and removed. The remaining residue was washed three times with each 200ml of 0.01M phosphate buffer solution (Na₂HPO₄ 1.15g, KH₂PO₄ 0.2g, KCl 0.2g, NaCl 8.766g, per liter, pH8.0) to obtain 600mg of immunoglobulin IgG having the purity of 96% or more.

### Example 13 : Therapeutic effect of the immunoglobulin on Pseudomonas aeruginosa infection

For immunoglobulins against Pseudomonas aeruginosa strains obtained in Example 12 above, their therapeutic effects on Pseudomonas aeruginosa infection were examined according to the following manner. Hereinafter, 10 mice were used in each group.

1.0-3.0x10⁶ cells of each of the pathogenic Pseudomonas aeruginosa strains of Fisher immunotypes 1, 2, 3, 4, 5, 6 and 7 were intraperitoneally injected into each mouse to cause Pseudomonas aeruginosa infection. Within 2 to 6 hours after the injection, the purified immunoglobulin for each strain was intravenously injected into each mouse in an amount of 0.1 to 2mg per mouse to examine the therapeutic effect thereof.

In the control group, 0.5ml of physiological saline for injection was intravenously injected instead of immunoglobulin. In this test, the immunoglobulins used were CFCPA 10142, CFCPA 20215, CFCPA 30720 and CFCPA 60534, or a mixture of these 4 kinds of immunoglobulins in the ratio of equivalent amounts.

As a result, the control group which received only physiological saline exhibited a lethality rate of 50% or more within 48 hours and 100% after 72 hours. Whereas the test group which received the corresponding immunoglobulin exhibited a survival rate of 80% or more after 72 hours. Accordingly, it could be demonstrated that the immunoglobulin is effective for the treatment of infection caused by pathogenic Pseudomonas aeruginosa strain having the corresponding Fisher immunotype. However, no single immunoglobulin had much of an effect on an infection caused by pathogenic Pseudomonas aeruginosa strains having Fisher immunotypes 4 and 5.

On the other hand, the mouse group which received the combination of 4 kinds of immunoglobulins in the ratio of 1:1:1:1 by weight showed superior therapeutic effect even on Fisher 4 and 5 types of Pseudomonas aeruginosa strains due to their cross-reactivity. Accordingly, the combined immunoglobulin composition according to the present invention can be used as a therapeutic agent effective for infection caused by Pseudomonas aeruginosa strains having all Fisher immunotypes. The results of this experiment are summarized in Tables 11 and 12.

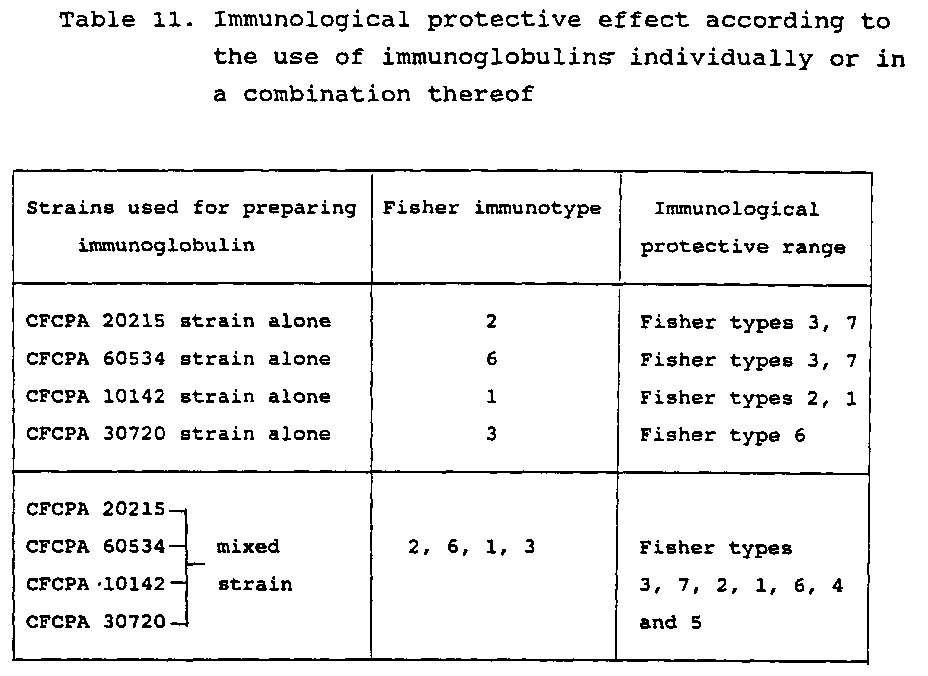

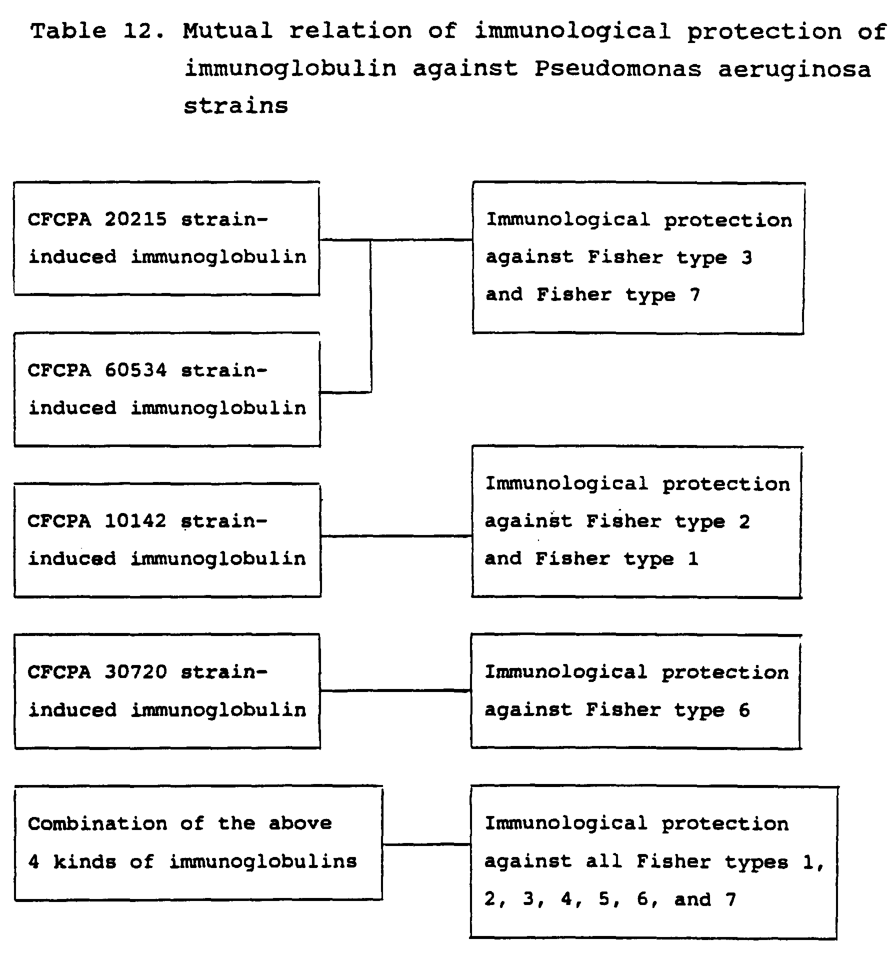

### Example 14 : Therapeutic effect of the combined immunoglobulins on Pseudomonas aeruginosa infection

4 kinds of attenuated Pseudomonas aeruginosa strains selected in Example 13, i.e. CFCPA 10142, CFCPA 20215, CFCPA 30720 and CFCPA 60534 were incubated and extracted according to the same procedures as Examples 5, 6 and 7 to obtain the combined composition of cell wall proteins which were then inoculated three times at intervals of one week in an amount of 1.5 to 2mg into goat according to the same manner as Example 13 to obtain the combined Pseudomonas aeruginosa immunoglobulins in a large quantity which was purified according to the known method [see, Practical Immunology, 3rd Ed., (1989), pp 292-294].

In order to determine the immunological protective effect and therapeutic effect of the purified and combined immunoglobulin obtained above on pathogenic Pseudomonas aeruginosa of various Fisher immunotypes, pathogenic Pseudomonas aeruginosa strains having each immunotype were inoculated into a mouse group (six groups : 20 mice per group) into which the combined immunoglobulin composition was previously injected three times, and another mouse group (six groups: 10 mice per group which received no immunoglobulin composition) in an amount of 1.0 to 3.0x10⁶ cells per mouse to observe the immunological protective effect of the combined immunoglobulin. In addition, in another mouse group (six groups: 10 mice per group) pathogenic Pseudomonas aeruginosa strain was first inoculated and after 2 to 6 hours the combined immunoglobulin composition obtained above was intravenously injected in an amount of 0.1 to 5mg per mouse weighing about 20 to 25g to observe the therapeutic effect of the immunoglobulin composition according to the present invention.

As a result thereof, it could be demonstrated that the combined immunoglobulin composition according to the present invention exhibits an immunological protective effect of 80%, or more, and a therapeutic effect of 75% or more (determined as the survival rate (%) after 7 days) while all the control groups have died within 3 days.

Accordingly, it can be determined that the combined immunoglobulin composition of the present invention can be used as a useful medicinal agent showing both excellent therapeutic effect and immunological protective effect.

### Example 15 : Formulation of immunoglobulins

The combination of cell wall proteins which were obtained by incubation and extraction of 4 kinds of attenuated Pseudomonas aeruginosa strains selected in Example 13, i.e. CFCPA 10142, CFCPA 20215, CFCPA 30720 and CFCPA 60534 according to the same manner as Examples 5, 6 and 7, was administered to male goat in the same manner as Example 14 to obtain the combined Pseudomonas aeruginosa immunoglobulin which was separated and purified, and then formulated using various pharmaceutically acceptable carriers so that 1 to 100mg of antibody immunoglobulin can be administered per kg of body weight. The formulated immunoglobulin was administered to mouse infected with Pseudomonas aeruginosa to determine the efficacy of the immunoglobulin depending on the kinds of carriers used the immunoglobulin. As a result of this, in the case of liquid formulation the immunoglobulin preparation with physiological saline for injection or phosphate buffer solution as a carrier exhibits a high efficacy and in the case of lyophilized formulation the use of mannitol, saccharose or lactose as a carrier provides the high efficacy of the immunoglobulin preparation. On the other hand, in a lyophilized formulation the use of human albumin together with Pseudomonas aeruginosa immunoglobulin according to the present invention lowers the efficacy of immunoglobulin and in the case of liquid formulation the use of aluminum hydroxide as a pharmaceutical carrier shows low efficacy. The test results are summarized in Table 13.

**Table 13**

| Efficacy of Pseudomonas aeruginosa immunoglobulin depending on pharmaceutical carriers | | |
|---|---|---|
| Formulation type | Constituents | Efficacy |
| Lyophilized formulation | Purified combined immunoglobulin composition + mannitol (5-20%) | ++++ |
| | Purified combined immunoglobulin composition + lactose (5-20%) | +++ |
| | Purified combined immunoglobulin composition + saccharose (5-20%) | +++ |
| | Purified combined immunoglobulin composition + human albumin (1-5%) | ++ |
| Liquid formulation | Purified combined immunoglobulin composition + physiological saline | ++++ |
| | Purified combined immunoglobulin composition + distilled water for injection | ++++ |
| | Purified combined immunoglobulin composition + phosphate buffer solution | ++++ |
| | Purified combined immunoglobulin composition + aluminum hydroxide + solvent | ++ |

### Example 16 : Efficacy of the formulated immunoglobulin composition

The formulated immunoglobulin with a phosphate buffer solut- ion, as identified to provide the high efficacy from the test result of Example 15, was examined to determine the efficacy as a prophylactic and therapeutic agent for a secondary cutaneous infection in laceration, burn, trauma, and the like. A mouse received a burn according to the burn test procedure [see, J. Infect. Dis. 131, 688-691, 1975]. The liquid preparation containing 0.5 to 1mg of combined Pseudomonas aeruginosa immunoglobulin per 1ml of phosphate buffer solution was formulated into a spray, which was then applied to the burn portion of the mouse in the test group to determine the effect thereof in comparison with that in the control group having no treatment with the immunoglobulin spray. As a result of this experiment, the mouse group treated with the immunoglobulin-phosphate buffer solution spray shows remarkably higher survival rate than the non-treated group. Accordingly, it can be demonstrated that the Pseudomonas aeruginosa immunoglobulin-containing preparation according to the present invention exhibits a superior protective effect and a superior therapeutic effect on Pseudomonas aeruginosa infection. The results of this experiment is described in the following Table 14.

**Table 14**

| Result of burning test | | | | | | |
|---|---|---|---|---|---|---|
| Treatment | Number of mouse | Pseudomonas aeruginosa inoculation | Survival rate | | | |
| | | | 24hrs. | 48hrs. | 72hrs. | 96hrs. or more |
| Burn (treated with immunoglobulin) | 20 | local inoculation | 20/20 | 19/20 | 16/20 | 16/20 |
| Burn (not treated with immunoglobulin) | 20 | local inoculation | 18/20 | 4/20 | 0/20 | - |
| Control group | 10 | no inoculation | 10/10 | 10/10 | 10/10 | 10/10 |

As described above, each attenuated Pseudomonas aeruginosa strain according to the present invention is considerably safe relative to the wild or pathogenic species. In addition, since the cell wall proteins thereof exhibit an excellent safety and cross-protective property and also a superior neutralizing antibody formation capacity, the cell wall proteins can be used not only as a vaccine for prophylaxis against Pseudomonas aeruginosa infection but also as an antibody inducer in the experimental animal to produce immunoglobulin which can used as a therapeutic agent for Pseudomonas aeruginosa infection. Accordingly, it can be seen that attenuated Pseudomonas aeruginosa strains according to the present invention are very useful microorganisms for preparing a prophylactic vaccine and a therapeutic agent for Pseudomonas aeruginosa infection.

## Claims

1. An attenuated Pseudomonas aeruginosa strain having an LD₅₀ of at least, 2.0 x 10⁷ cells in mice and which is obtainable by isolating Pseudomonas aeruginosa in a pure state according to immunotype from patients infected with Pseudomonas aeruginosa and subjecting the isolated strain to repeat purification to attenuate the strain, said Pseudomonas aeruginosa strain being selected within the group consisting in Pseudomonas aeruginosa strains having the Korean Federation of Culture Collection accession numbers KCCM 10029 (CFCPA 10142), KCCM 10030 (CFCPA 20215), KCCM 10031 (CFCPA 30720), KCCM 10032 (CFCPA 40057), KCCM 10033 (CFCPA 50243), KCCM 10034 (CFCPA 60534) and KCCM 10035 (CFCPA 70018).

2. The Pseudomonas aeruginosa strain according to claim 1 having Korean Federation of Culture Collection accession number KCCM 10029 (CFCPA 10142).

3. The Pseudomonas aeruginosa strain according to claim 1 having Korean Federation of Culture Collection accession number KCCM 10030 (CFCPA 20215).

4. The Pseudomonas aeruginosa strain according to claim 1 having Korean Federation of Culture Collection accession number KCCM 10031 (CFCPA 30720).

5. The Pseudomonas aeruginosa strain according to claim 1 having Korean Federation of Culture Collection accession number KCCM 10032 (CFCPA 40057).

6. The Pseudomonas aeruginosa strain according to claim 1 having Korean Federation of Culture Collection accession number KCCM 10033 (CFCPA 50243).

7. The Pseudomonas aeruginosa strain according to claim 1 having Korean Federation of Culture Collection accession number KCCM 10034 (CFCPA 60534).

8. The Pseudomonas aeruginosa strain according to claim 1 having Korean Federation of Culture Collection accession number KCCM 10035 (CFCPA 70018).

9. Use of at least one Pseudomonas aeruginosa strain selected from the group consisting of:
- Pseudomonas aeruginosa strain having Korean Federation of Culture Collection accession number KCCM 10029 (CFCPA 10142),
- Pseudomonas aeruginosa strain having Korean Federation of Culture Collection accession number KCCM 10030 (CFCPA 20215),
- Pseudomonas aeruginosa strain having Korean Federation of Culture Collection accession number KCCM 10031 (CFCPA 30720),
- Pseudomonas aeruginosa strain having Korean Federation of Culture Collection accession number KCCM 10032 (CFCPA 40057),
- Pseudomonas aeruginosa strain having Korean Federation of Culture Collection accession number KCCM 10033 (CFCPA 50243),
- Pseudomonas aeruginosa strain having Korean Federation of Culture Collection accession number KCCM 10034 (CFCPA 60534),
- Pseudomonas aeruginosa strain having Korean Federation .of Culture Collection accession number KCCM 10035 (CFCPA 70018), in preparation of a vaccine for immunization against Pseudomonas aeruginosa infection.

10. A non-pathogenic vaccine for inducing an immune response against a disease caused by Pseudomonas aeruginosa comprising a mixture of cell wall proteins having a molecular weight range of 10,000 to 100,000 derived from at least one attenuated Pseudomonas aeruginosa strain selected from the group consisting of:
- Pseudomonas aeruginosa strain having Korean Federation of Culture Collection accession number KCCM 10029 (CFCPA 10142),
- Pseudomonas aeruginosa strain having Korean Federation of Culture collection accession number KCCM 10030 (CFCPA 20215),
- Pseudomonas aeruginosa strain having Korean Federation of Culture Collection accession number KCCM 10031 (CFCPA 30720),
- Pseudomonas aeruginosa strain having Korean Federation of Culture Collection accession number KCCM 10032 (CFCPA 40057),
- Pseudomonas aeruginosa strain having Korean Federation of Culture Collection accession number KCCM 10033 (CFCPA 50243),
- Pseudomonas aeruginosa strain having Korean Federation of Culture Collection accession number KCCM 10034 (CFCPA 60534), and
- Pseudomonas aeruginosa strain having Korean Federation of Culture Collection accession number KCCM 10035 (CFCPA 70018),
to induce an immune response to prevent subsequent disease caused by Pseudomonas aeruginosa.

11. The vaccine according to claim 10, wherein the cell wall proteins are derived from at least three attenuated Pseudomonas aeruginosa strains selected from the group of: CFCPA 10142, CFCPA 20215, CFCPA 30720, CFCPA 40057, CFCPA 50243, CFCPA 60534 and CFCPA 70018.

12. The vaccine according to claim 11, wherein the attenuated Pseudomonas aeruginosa strains consist of: CFCPA 10142, CFCPA 20215 and CFCPA 30720.

13. The vaccine according to claim 11, wherein the attenuated Pseudomonas aeruginosa strains are selected from the group consisting of: CFCPA 10142, CFCPA 20215, CFCPA 30720 and CFCPA 60534.

14. The vaccine according to claim 11, wherein the attenuated Pseudomonas aeruginosa strains are CFCPA 10142, CFCPA 20215, CFCPA 30720, CFCPA 40057, CFCPA 50243, CFCPA 60534 and CFCPA 70018.

15. The vaccine according to any one of claims 10 to 14, wherein the cell wall proteins from each of the attenuated Pseudomonas aeruginosa strains present are present in equivalent amounts.

16. The vaccine according to claim 10, further including pharmaceutically acceptable excipients.

17. A process for preparing a non-pathogenic vaccine for immunization against Pseudomonas aeruginosa infection, comprising :
a) collecting Pseudomonas aeruginosa from patients infected with Pseudomonas aeruginosa;
b) selecting and isolating at least one species of Pseudomonas aeruginosa in a pure state;
c) incubating each pure isolated Pseudomonas aeruginosa in a first culture medium;
d) attenuating each pure incubated strain by repeat injections into experimental animals and selecting the most attenuated strain for each Pseudomonas aeruginosa strain attenuated;
e) incubating each attenuated Pseudomonas aeruginosa strain in a second culture medium;
f) separating the attenuated Pseudomonas aeruginosa from the culture medium;
g) treating the separated Pseudomonas aeruginosa with an organic solvent to inactivate and remove cell wall lipid components;
h) extracting the cell wall protein from the treated Pseudomonas aeruginosa while simultaneously determining whether the cells of Pseudomonas aeruginosa are being destroyed by ascertaining the presence of lactate dehydrogenase or hexokinase as a cytoplasmic marker enzyme;
i) subjecting the extracted cell wall protein to ultrafiltration to select only those cell wall proteins having the molecular weight ranging from 10,000 to 100,000;
j) ultracentrafuging the cell wall protein to further remove any lipopolysaccharide and any bacterial substances;
k) recovering the selected cell wall proteins in a pure state; and
l) preparing a vaccine using the selected cell wall proteins as an antigenic substance to induce immunization.

18. The process of claim 17, wherein the extraction is performed by placing the treated Pseudomonas aeruginosa cells in a buffer solution.

19. The process of claim 18, wherein the buffer solution, is selected from the group consisting of a phosphate buffer solution and a tris buffer solution.

20. The process of claim 17, wherein the extraction of the cell wall proteins is performed by standing the bacterial mass or acting on the treated Pseudomonas aeruginosa cells with a mixer or homogenizer.

21. The process of claim 20, wherein the mixer is operated at 100 to 2000 rpm at 4°C.

22. The process of claim 17, wherein the extraction is performed 5 to 6 times.

23. The process of claim 17, wherein the organic solvent is selected from the group consisting of acetone, chloroform, ethanol, butanol and most preferably the organic solvent is acetone.

24. The process of claim 17, wherein the Pseudomonas aeruginosa strains isolated include KCCM 10029 (CFCPA 10142), KCCM 10030 (CFCPA 20215), KCCM 10031 (CFCPA 30720), KCCM 10032 (CFCPA 40057), KCCM 10033 (CFCPA 50243), KCCM 10034 (CFCPA 60534) and KCCM 10035 (CFCPA 70018).

25. The process of claim 17 wherein the second culture medium is selected from the group consisting of tryptic soy broth and a culture medium comprising glucose (30 g/l), peptone (15 g/l), MgS0₄ (0.5 g/l), CaCO₃ (5 g/l), KH₂PO₄ (1 g/l), FeS0₄ (5 mg/l), CuS0₄ (5 mg/l) and ZnS0₄ (5 mg/l).

26. The process of claim 17, wherein the incubation of the attenuated Pseudomonas aeruginosa conditions comprise : a temperature of 37°C, an aeration rate 1 vvm (volume/volume minute) and a seed volume of 5% v/v of the culture medium solution, with the incubation carried out under a stirring rate of 100 rpm for the first 2 hours and then at 600 rpm for the following 10 to 20 hours, preferably for 12 to 16 hours.

27. A therapeutic agent for treating Pseudomonas aeruginosa infection in a patient manifesting the infection, said agent comprises at least one immunoglobulin specific against at least one Pseudomonas aeruginosa strain, wherein said immunoglobulin is induced by injection into a suitable host cell wall proteins having a molecular weight range of 10,000 to 100,000 derived from at least one attenuated Pseudomonas aeruginosa strain selected from the group consisting of :
- Pseudomonas aeruginosa strain having Korean Federation of Culture Collection accession number KCCM 10029 (CFCPA 10142),
- Pseudomonas aeruginosa strain having Korean Federation of Culture Collection accession number KCCM 10030 (CFCPA 20215),
- Pseudomonas aeruginosa strain having Korean Federation of Culture Collection accession number KCCM 10031 (CFCPA 30720),
- Pseudomonas aeruginosa strain having Korean Federation of Culture Collection accession number KCCM 10032 (CFCPA 40057),
- Pseudomonas aeruginosa strain having Korean Federation of Culture Collection accession number KCCM 10033 (CFCPA 50243),
- Pseudomonas aeruginosa strain having Korean Federation of Culture Collection accession number KCCM 10034 (CFCPA 60534), and
- Pseudomonas aeruginosa strain having Korean Federation of Culture Collection accession number KCCM 10035 (CFCPA 70018), in an amount sufficient to induce an immune response in said host to initiate the production of said immunoglobulin to, at least lessen the untoward effects of the disease caused by Pseudomonas aeruginosa.

28. The therapeutic agent of claim 27 which contains an immunoglobulin against each of the Pseudomonas aeruginosa strains corresponding to Fisher immunotypes 1, 2, 3, 4, 5, 6 and 7.

29. The therapeutic agent of claim 27, containing immunoglobulins against any four of the Pseudomonas aeruginosa strains corresponding to Fisher immunotypes 1, 2, 3, 4, 5, 6 and 7.

30. The therapeutic agent of claim 27, containing immunoglobulins against any three of the Pseudomonas aeruginosa strains corresponding to Fisher immunotypes 1, 2, 3, 4, 5, 6 and 7.

31. The therapeutic agent of claim 27 in a lyophilized form.

32. The therapeutic agent of claim 31 further including a suitable pharmaceutically acceptable carrier selected from the group consisting of mannitol, lactose, saccharose and human albumin.

33. The therapeutic agent of claim 27 in a liquid form and further including suitable pharmaceutically acceptable carriers.

34. The therapeutic agent of claim 27 prepared in a pharmaceutical dosage form as an injection, tablet, capsule, eye drop, spray or ointment.

## Patentansprüche

1. Abgeschwächter Pseudomonas aeruginosa Stamm mit einer LD₅₀ von mindestens 2,0 x 10⁷ Zellen in Mäusen, der durch Isolierung von Pseudomonas aeruginosa in bezüglich des Immunotyps reinem Zustand von mit Pseudomonas aeruginosa infizierten Patienten und Unterziehen des isolierten Stammes einer wiederholten Aufreinigung zur Schwächung des Stammes erhältlich ist, wobei der Pseudomonas aeruginosa Stamm aus der Gruppe ausgewählt ist, die aus den Pseudomonas aeruginosa Stämmen mit den folgenden Zugangsnummern der Koreanischen Vereinigung für Kultursammlung (Korean Federation of Culture Collection) besteht: KCCM 10029 (CFCPA 10142), KCCM 10030 (CFCPA 20215), KCCM 10031 (CFCPA 30720), KCCM 10032 (CFCPA 40057), KCCM 10033 (CFCPA 50243), KCCM 10034 (CFCPA 60534) und KCCM 10035 (CFCPA 70018).

2. Pseudomonas aeruginosa Stamm gemäß Anspruch 1 mit der Zugangsnummer der Koreanischen Vereinigung für Kultursammlung KCCM 10029 (CFCPA 10142).

3. Pseudomonas aeruginosa Stamm gemäß Anspruch 1 mit der Zugangsnummer der Koreanischen Vereinigung für Kultursammlung KCCM 10030 (CFCPA 20215).

4. Pseudomonas aeruginosa Stamm gemäß Anspruch 1 mit der Zugangsnummer der Koreanischen Vereinigung für Kultursammlung KCCM 10031 (CFCPA 30720).

5. Pseudomonas aeruginosa Stamm gemäß Anspruch 1 mit der Zugangsnummer der Koreanischen Vereinigung für Kultursammlung KCCM 10032 (CFCPA 40057).

6. Pseudomonas aeruginosa Stamm gemäß Anspruch 1 mit der Zugangsnummer der Koreanischen Vereinigung für Kultursammlung KCCM 10033 (CFCPA 50243).

7. Pseudomonas aeruginosa Stamm gemäß Anspruch 1 mit der Zugangsnummer der Koreanischen Vereinigung für Kultursammlung KCCM 10034 (CFCPA 60534).

8. Pseudomonas aeruginosa Stamm gemäß Anspruch 1 mit der Zugangsnummer der Koreanischen Vereinigung für Kultursammlung KCCM 10035 (CFCPA 70018).

9. Verwendung von mindestens einem Pseudomonas aeruginosa Stamm, der aus der Gruppe ausgewählt ist, die besteht aus:
- Pseudomonas aeruginosa Stamm mit der Zugangsnummer der Koreanischen Vereinigung für Kultursammlung KCCM 10029 (CFCPA 10142),
- Pseudomonas aeruginosa Stamm mit der Zugangsnummer der Koreanischen Vereinigung für Kultursammlung KCCM 10030 (CFCPA 20215),
- Pseudomonas aeruginosa Stamm mit der Zugangsnummer der Koreanischen Vereinigung für Kultursammlung KCCM 10031 (CFCPA 30720),
- Pseudomonas aeruginosa Stamm mit der Zugangsnummer der Koreanischen Vereinigung für Kultursammlung KCCM 10032 (CFCPA 40057),
- Pseudomonas aeruginosa Stamm mit der Zugangsnummer der Koreanischen Vereinigung für Kultursammlung KCCM 10033 (CFCPA 50243),
- Pseudomonas aeruginosa Stamm mit der Zugangsnummer der Koreanischen Vereinigung für Kultursammlung KCCM 10034 (CFCPA 60534),
- Pseudomonas aeruginosa Stamm mit der Zugangsnummer der Koreanischen Vereinigung für Kultursammlung KCCM 10035 (CFCPA 70018) zur Herstellung eines Impfstoffes für die Immunisierung gegen Pseudomonas aeruginosa Infektion.

10. Nicht pathogener Impfstoff zur Erzeugung einer Immunantwort gegen eine von Pseudomonas aeruginosa hervorgerufene Erkrankung, umfassend eine Mischung aus Zellwandproteinen mit einem Molekulargewichtsbereich von 10.000 bis 100.000, die von mindestens einem geschwächten Pseudomonas aeruginosa Stamm abgeleitet sind, der aus der Gruppe ausgewählt ist, die besteht aus:
- Pseudomonas aeruginosa Stamm mit der Zugangsnummer der Koreanischen Vereinigung für Kultursammlung KCCM 10029 (10142),
- Pseudomonas aeruginosa Stamm mit der Zugangsnummer der Koreanischen Vereinigung für Kultursammlung KCCM 10030 (CFCPA 20215),
- Pseudomonas aeruginosa Stamm mit der Zugangsnummer der Koreanischen Vereinigung für Kultursammlung KCCM 10031 (CFCPA 30720),
- Pseudomonas aeruginosa Stamm mit der Zugangsnummer der Koreanischen Vereinigung für Kultursammlung KCCM 10032 (CFCPA 40057),
- Pseudomonas aeruginosa Stamm mit der Zugangsnummer der Koreanischen Vereinigung für Kultursammlung KCCM 10033 (CFCPA 50243),
- Pseudomonas aeruginosa Stamm mit der Zugangsnummer der Koreanischen Vereinigung für Kultursammlung KCCM 10034 (CFCPA 60534),
- Pseudomonas aeruginosa Stamm mit der Zugangsnummer der Koreanischen Vereinigung für Kultursammlung KCCM 10035 (CFCPA 70018),
um eine Immunantwort zu induzieren und eine nachfolgende von Pseudomonas aeruginosa verursachte Erkrankung zu verhindern.

11. Impfstoff gemäß Anspruch 10, wobei die Zellwandproteine von mindestens 3 geschwächten Pseudomonas aeruginosa Stämmen abgeleitet sind, die aus der folgenden Gruppe ausgewählt sind: CFCPA 10142, CFCPA 20215, CFCPA 30720, CFCPA 40057, CFCPA 50243, CFCPA 60534 und CFCPA 70018.

12. Impfstoff gemäß Anspruch 11, wobei die geschwächten Pseudomonas aeruginosa Stämme aus CFCPA 10142, CFCPA 20215 und CFCPA 30720 bestehen.

13. Impfstoff gemäß Anspruch 11, wobei die geschwächten Pseudomonas aeruginosa Stämme aus der Gruppe ausgewählt sind, die besteht aus: CFCPA 10142, CFCPA 20215, CFCPA 30720 und CFCPA 60534.

14. Impfstoff gemäß Anspruch 11, wobei die geschwächten Pseudomonas aeruginosa Stämme CFCPA 10142, CFCPA 20215, CFCPA 30720, CFCPA 40057, CFCPA 50243, CFCPA 60534 und CFCPA 70018 sind.

15. Impfstoff gemäß mindestens einem der Ansprüche 10 bis 14, wobei die Zellwandproteine aus jedem der vorliegenden geschwächten Pseudomonas aeruginosa Stämme in äquivalenten Mengen vorliegen.

16. Impfstoff gemäß Anspruch 10, der des weiteren einen pharmazeutisch akzeptablen Arzneimittelträger einschließt.

17. Verfahren zur Herstellung eines nicht-pathogenen Impfstoffs zur Immunisierung gegen eine Pseudomonas aeruginosa Infektion, umfassend:
a) Gewinnen von Pseudomonas aeruginosa von mit Pseudomonas aeruginosa infizierten Patienten;
b) Selektieren und Isolieren mindestens einer Spezies von Pseudomonas aeruginosa in reinem Zustand;
c) Inkubieren jedes reinen isolieren Pseudomonas aeruginosa in einem ersten Kulturmedium;
d) Aufreinigen jedes in reiner Form inkubierten Stammes durch wiederholte Injektionen in Versuchstiere und Auswählen des am stärksten geschwächten Stammes für jeden geschwächten Pseudomonas aeruginosa Stamm;
e) Inkubieren jedes geschwächten Pseudomonas aeruginosa Stammes in einem zweiten Kulturmedium;
f) Separieren des geschwächten Pseudomonas aeruginosa aus dem Kulturmedium;
g) Behandeln des abgetrennten Pseudomonas aeruginosa mit einem organischen Lösungsmittel zum Inaktivieren und Entfernen von Lipidkomponenten der Zellwand;
h) Extrahieren der Zellwandproteine aus dem behandeltem Pseudomonas aeruginosa bei gleichzeitiger Bestimmung, ob die Pseudomonas aeruginosa Zellen zerstört worden sind, indem das Vorliegen von Lactatdehydrogenase oder Hexokinase als cytoplasmisches Markerenzym sichergestellt wird;
i) Ultrafiltration des extrahierten Zellwandproteins, um nur die Zellwandproteine auszuwählen, die ein Molekulargewicht im Bereich von 10.000 bis 100.000 aufweisen; j) Ultrazentrifugation des Zellwandproteins, um jedes Lipopo
lysaccharid und alle bakteriellen Substanzen weiter zu entfernen;
k) Gewinnen des ausgewählten Zellwandproteins in reinem Zustand und
l) Herstellen des Impfstoffs unter Verwendung der ausgewählten Zellwandproteine als antigene Substanz zur Indizierung einer Immunisierung.

18. Verfahren nach Anspruch 17, wobei die Extraktion durch Einbringen der behandelten Pseudomonas aeruginosa Zellen in eine Pufferlösung durchgeführt wird.

19. Verfahren nach Anspruch 18, wobei die Pufferlösung aus der Gruppe ausgewählt ist, die aus einer Phosphat-Pufferlösung und einer tris-Pufferlösung besteht.

20. Verfahren nach Anspruch 17, wobei die Extraktion der Zellwandproteine durch stehen lassen der bakteriellen Masse oder durch Einwirken auf die behandelten Pseudomonas aeruginosa Zellen mit einem Mixer oder Homogenisator durchgeführt wird.

21. Verfahren nach Anspruch 20, wobei der Mixer bei 100 bis 2000 UpM bei 4°C eingesetzt wird.

22. Verfahren nach Anspruch 17, wobei die Extraktion 5 bis 6 mal durchgeführt wird.

23. Verfahren nach Anspruch 17, wobei das organische Lösungsmittel aus der Gruppe ausgewählt ist, die aus Azeton, Chloroform, Ethanol und Butanol besteht, wobei das am stärksten bevorzugte organische Lösungsmittel Azeton ist.

24. Verfahren nach Anspruch 17, wobei die isolierten Pseudomonas aeruginosa Stämme KCCM 10029 (CFCPA 10142), KCCM 10030 (CFCPA 20215), KCCM 10031 (CFCPA 30720), KCCM 10032 (CFCPA 40057), KCCM 10033 (CFCPA 50243), KCCM 1034 (CFCPA 60534) und KCCM 10035 (CFCPA 70018) einschließen.

25. Verfahren nach Anspruch 17, wobei das zweite Kulturmedium aus der Gruppe ausgewählt ist, die besteht aus: Tryptischer Sojanährlösung und einem Kulturmedium, umfassend Glucose (30g/l), Pepton (15g/l), MgSO₄ (0,5 g/l), CaCO₃ (5 g/l), KH₂PO₄ (1 g/l), FeSO₄ (5 mg/l), CuSO₄ (5 mg/l) und ZnSO₄ (5 mg/l).

26. Verfahren nach Anspruch 17, wobei die Inkubationsbedingungen des geschwächten Pseudomonas aeruginosa umfassen: eine Temperatur von 37°C, eine Belüfungsrate vom 1 vvm (Volumen/Volumen Minute) und ein Aussähvolumen von 5% v/v bezogen auf die Kulturmedienlösung, wobei die Inkubation für die ersten beiden Stunden bei einer Rührrate von 100 UpM und für die folgenden 10 bis 20 Stunden, vorzugsweise 12 bis 16 Stunden, bei 600 UpM durchgeführt wird.

27. Heilmittel zur Behandlung einer Pseudomonas aeruginosa Infektion bei einem Patienten mit manifester Infektion, wobei das Heilmittel mindestens ein Immunoglobulin umfaßt, daß für mindestens einen Pseudomonas aeruginosa Stamm spezifisch ist, wobei das Immunoglobulin durch Injektion in einen geeigneten Wirt von Zellwattproteinen eines Molekulargewichtsbereich von 10.000 bis 100.000 induziert wird, die von mindestens einem geschwächten Pseudomonas aeruginosa Stamm abgeleitet sind, der aus der Gruppe ausgewählt ist, die besteht aus:
- Pseudomonas aeruginosa Stamm mit der Zugangsnummer der Koreanischen Vereinigung für Kultursammlung KCCM 10029 (CFCPA 10142),
- Pseudomonas aeruginosa Stamm mit der Zugangsnummer der Koreanischen Vereinigung für Kultursammlung KCCM 10030 (CFCPA 20215),
- Pseudomonas aeruginosa Stamm mit der Zugangsnummer der Koreanischen Vereinigung für Kultursammlung KCCM 10031 (CFCPA 30720),
- Pseudomonas aeruginosa Stamm mit der Zugangsnummer der Koreanischen Vereinigung für Kultursammlung KCCM 10032 (CFCPA 40057),
- Pseudomonas aeruginosa Stamm mit der Zugangsnummer der Koreanischen Vereinigung für Kultursammlung KCCM 10033 (CFCPA 50243),
- Pseudomonas aeruginosa Stamm mit der Zugangsnummer der Koreanischen Vereinigung für Kultursammlung KCCM 10034 (CFCPA 60534),
- Pseudomonas aeruginosa Stamm mit der Zugangsnummer der Koreanischen Vereinigung für Kultursammlung KCCM 10035 (CFCPA 70018), in einer zum Indizieren einer Immunantwort in dem Wirt ausreichenden Menge, um die Produktion des Immunoglobulins zu iniziieren und die unerwünschten Effekte der von Pseudomonas aeruginosa verursachten Erkrankung zumindest zu vermindern.

28. Heilmittel nach Anspruch 27, das ein Immunoglobulin gegen jeden der Pseudomonas aeruginosa Stämme enthält, die den Fisher Immunotypen 1, 2, 3, 4, 5, 6 und 7 entsprechen.

29. Heilmittel nach Anspruch 27, das Immunoglobuline gegen alle vier Pseudomonas aeruginosa Stämme enthält, die den Fisher Immunotypen 1, 2, 3, 4, 5, 6 und 7 entsprechen.

30. Heilmittel nach Anspruch 27, das Immunoglobuline gegen jeden der drei Pseudomonas aeruginosa Stämme enthält, die den Fisher Immunotypen 1, 2, 3, 4, 5, 6 und 7 entsprechen.

31. Heilmittel nach Anspruch 27 in lyophilizierter Form.

32. Heilmittel nach Anspruch 31, das darüber hinaus einen geeigneten pharmazeutisch akzeptablen Träger einschließt, der aus der aus Mannitol, Laktose, Saccharose und humanen Albumin bestehenden Gruppe ausgewählt ist.

33. Heilmittel nach Anspruch 27 in flüssiger Form, das darüber hinaus geeignete pharmazeutische akzeptable Träger einschließt.

34. Heilmittel nach Anspruch 27, hergestellt in Form einer pharmazeutischen Dosierung als Injektion, Tablette, Kapsel, Augentropfen, Spray oder Salbe.

## Revendications

1. Souche atténuée de *Pseudomonas aeruginosa* ayant une DL₅₀ d'au moins 2,0 x 10⁷ cellules chez les souris et qu'on peut obtenir par isolement de *Pseudomonas aeruginosa* à l'état pur, selon l'immunotype, chez des patients infectés par le *Pseudomonas aeruginosa* et en soumettant la souche isolée à une purification répétée pour atténuer ladite souche, ladite souche de *Pseudomonas aeruginosa* étant choisie dans l'ensemble constitué par des souches de *Pseudomonas aeruginosa* ayant les numéros d'accès de la Fédération Coréenne de Collection de Cultures KCCM 10029 (CFCPA 10142), KCCM 10030 (CFCPA 20215), KCCM 10031 (CFCPA 30720), KCCM 10032 (CFCPA 40057), KCCM 10033 (CFCPA 50243), KCCM 10034 (CFCPA 60534) et KCCM 10035 (CFCPA 70018).

2. Souche de *Pseudomonas aeruginosa* selon la revendication 1, ayant le numéro d'accès de la Fédération Coréenne de Collection de Cultures KCCM 10029 (CFCPA 10142).

3. Souche de *Pseudomonas aeruginosa* selon la revendication 1, ayant le numéro d'accès de la Fédération Coréenne de Collection de Cultures KCCM 10030 (CFCPA 20215).

4. Souche de *Pseudomonas aeruginosa* selon la revendication 1, ayant le numéro d'accès de la Fédération Coréenne de Collection de Cultures KCCM 10031 (CFCPA 30720).

5. Souche de *Pseudomonas aeruginosa* selon la revendication 1, ayant le numéro d'accès de la Fédération Coréenne de Collection de Cultures KCCM 10032 (CFCPA 40057).

6. Souche de *Pseudomonas aeruginosa* selon la revendication 1, ayant le numéro d'accès de la Fédération Coréenne de Collection de Cultures KCCM 10033 (CFCPA 50243).

7. Souche de *Pseudomonas aeruginosa* selon la revendication 1, ayant le numéro d'accès de la Fédération Coréenne de Collection de Cultures KCCM 10034 (CFCPA 60534).

8. Souche de *Pseudomonas aeruginosa* selon la revendication 1, ayant le numéro d'accès de la Fédération Coréenne de Collection de Cultures KCCM 10035 (CFCPA 70018).

9. Utilisation d'au moins une souche de *Pseudomonas aeruginosa* choisie dans l'ensemble constitué par :
- souche de *Pseudomonas aeruginosa* ayant le numéro d'accès de la Fédération Coréenne de Collection de Cultures KCCM 10029 (CFCPA 10142),
- souche de *Pseudomonas aeruginosa* ayant le numéro d'accès de la Fédération Coréenne de Collection de Cultures KCCM 10030 (CFCPA 20215),
- souche de *Pseudomonas aeruginosa* ayant le numéro d'accès de la Fédération Coréenne de Collection de Cultures KCCM 10031 (CFCPA 30720),
- souche de *Pseudomonas aeruginosa* ayant le numéro d'accès de la Fédération Coréenne de Collection de Cultures KCCM 10032 (CFCPA 40057),
- souche de *Pseudomonas aeruginosa* ayant le numéro d'accès de la Fédération Coréenne de Collection de Cultures KCCM 10033 (CFCPA 50243),
- souche de *Pseudomonas aeruginosa* ayant le numéro d'accès de la Fédération Coréenne de Collection de Cultures KCCM 10034 (CFCPA 60534),
- souche de *Pseudomonas aeruginosa* ayant le numéro d'accès de la Fédération Coréenne de Collection de Cultures KCCM 10035 (CFCPA 70018);
pour la préparation d'un vaccin pour une immunisation contre une infection par *Pseudomonas aeruginosa*.

10. Vaccin non pathogène pour induire une réponse immune vis-à-vis d'une maladie causée par *Pseudomonas aeruginosa*, comprenant un mélange de protéines de parois cellulaires ayant un poids moléculaire compris entre 10 000 et 100 000, provenant d'au moins une souche atténuée de *Pseudomonas aeruginosa* choisie dans l'ensemble constitué par :
- souche de *Pseudomonas aeruginosa* ayant le numéro d'accès de la Fédération Coréenne de Collection de Cultures KCCM 10029 (CFCPA 10142),
- souche de *Pseudomonas aeruginosa* ayant le numéro d'accès de la Fédération Coréenne de Collection de Cultures KCCM 10030 (CFCPA 20215),
- souche de *Pseudomonas aeruginosa* ayant le numéro d'accès de la Fédération Coréenne de Collection de Cultures KCCM 10031 (CFCPA 30720),
- souche de *Pseudomonas aeruginosa* ayant le numéro d'accès de la Fédération Coréenne de Collection de Cultures KCCM 10032 (CFCPA 40057),
- souche de *Pseudomonas aeruginosa* ayant le numéro d'accès de la Fédération Coréenne de Collection de Cultures KCCM 10033 (CFCPA 50243),
- souche de *Pseudomonas aeruginosa* ayant le numéro d'accès de la Fédération Coréenne de Collection de Cultures KCCM 10034 (CFCPA 60534) et
- souche de *Pseudomonas aeruginosa* ayant le numéro d'accès de la Fédération Coréenne de Collection de Cultures KCCM 10035 (CFCPA 70018),
pour induire une réponse immune pour empêcher une maladie subséquente causée par *Pseudomonas aeruginosa*.

11. Vaccin selon la revendication 10, dans lequel les protéines de parois cellulaires proviennent d'au moins trois souches atténuées de *Pseudomonas aeruginosa* choisies dans l'ensemble constitué par CFCPA 10142, CFCPA 20215, CFCPA 30720, CFCPA 40057, CFCPA 50243, CFCPA 60534 et CFCPA 70018.

12. Vaccin selon la revendication 11, dans lequel les souches atténuées de *Pseudomonas aeruginosa* sont constituées par CFCPA 10142, CFCPA 20215 et CFCPA 30720.

13. Vaccin selon la revendication 11, dans lequel les souches atténuées de *Pseudomonas aeruginosa* sont choisies dans l'ensemble constitué par CFCPA 10142, CFCPA 20215, CFCPA 30720 et CFCPA 60534.

14. Vaccin selon la revendication 11, dans lequel les souches atténuées de *Pseudomonas aeruginosa* sont CFCPA 10142, CFCPA 20215, CFCPA 30720, CFCPA 40057, CFCPA 50243, CFCPA 60534 et CFCPA 70018.

15. Vaccin selon l'une quelconque des revendications 10 à 14, dans lequel les protéines de parois cellulaires provenant de chacune des souches atténuées de *Pseudomonas aeruginosa* sont présentes en des quantités équivalentes.

16. Vaccin selon la revendication 10, comprenant en outre des excipients pharmaceutiquement acceptables.

17. Procédé de préparation d'un vaccin non pathogène pour une immunisation contre une infection par *Pseudomonas aeruginosa*, comprenant les étapes consistant à :
a) recueillir du *Pseudomonas aeruginosa* sur des patients infectés par *Pseudomonas aeruginosa ;*
b) sélectionner et isoler au moins une espèce de *Pseudomonas aeruginosa* à l'état pur ;
c) faire incuber chaque *Pseudomonas aeruginosa* pur isolé dans un premier milieu de culture ;
d) atténuer chaque souche pure incubée au moyen d'injections répétées à des animaux expérimentaux et sélectionner la souche la plus atténuée pour chaque souche atténuée de *Pseudomonas aeruginosa* ;
e) faire incuber chaque souche atténuée de *Pseudomonas aeruginosa* dans un second milieu de culture ;
f) séparer le *Pseudomonas aeruginosa* atténué du milieu de culture ;
g) traiter le *Pseudomonas aeruginosa* séparé avec un solvant organique pour inactiver et éliminer les composants lipidiques des parois cellulaires ;
h) extraire les protéines des parois cellulaires à partir du *Pseudomonas aeruginosa* traité tout en déterminant simultanément si les cellules de *Pseudomonas aeruginosa* sont détruites en s'assurant de la présence de lactatedéshydrogénase ou d'hexokinase on tant qu'enzyme de marquage cytoplasmique ;
i) soumettre les protéines extraites des parois cellulaires à une ultrafiltration pour sélectionner seulement les protéines de parois cellulaires dont le poids moléculaire est compris entre 10 000 et 100 000 ;
j) soumettre les protéines des parois cellulaires à une ultracentrifugation pour éliminer de façon supplémentaire tout lipopolysaccharide et toutes les substances bactériennes ;
k) recueillir les protéines sélectionnées de parois cellulaires à l'état pur ; et
l) préparer un vaccin en utilisant les protéines sélectionnées de parois cellulaires en tant que substance antigénique pour induire une immunisation.

18. Procédé selon la revendication 17, dans lequel on effectue l'extraction en plaçant les cellules traitées de *Pseudomonas aeruginosa* dans une solution tampon.

19. Procédé selon la revendication 18, dans lequel on choisit la solution tampon dans l'ensemble constitué par une solution tampon de phosphate et une solution de tampon Tris.

20. Procédé selon la revendication 17, dans lequel on effectue l'extraction des protéines des parois cellulaires en laissant reposer la masse bactérienne ou en agissant sur les cellules traitées de *Pseudomonas aeruginosa* avec un malaxeur ou un homogénéisateur.

21. Procédé selon la revendication 20, dans lequel on actionne le malaxeur à une vitesse de 100 à 2000 tr/min à 4°C.

22. Procédé selon la revendication 17, dans lequel on effectue l'extraction 5 ou 6 fois.

23. Procédé selon la revendication 17, dans lequel on choisit le solvant organique dans l'ensemble constitué par l'acétone, le chloroforme, l'éthanol et le butanol, et de la façon la plus appréciée, le solvant organique est de l'acétone.

24. Procédé selon la revendication 17, dans lequel les souches isolées de *Pseudomonas aeruginosa* comprennent KCCM 10029 (CFCPA 10142), KCCM 10030 (CFCPA 20215), KCCM 10031 (CFCPA 30720), KCCM 10032 (CFCPA 40057), KCCM 10033 (CFCPA 50243), KCCM 10034 (CFCPA 60534) et KCCM 10035 (CFCPA 70018).

25. Procédé selon la revendication 17, dans lequel on choisit le second milieu de culture dans l'ensemble constitué par un bouillon trypsique de soja et un milieu de culture comprenant du glucose (30 g/l), de la peptone (15 g/l), du MgSO₄ (0,5 g/l), du CaCO₃ (5 g/l), du KH₂PO₄ (1 g/l), du FeSO₄ (5 mg/l), du CuSO₄ (5 mg/l) et du ZnSO₄ (5 mg/l).

26. Procédé selon la revendication 17, dans lequel les conditions d'incubation du *Pseudomonas aeruginosa* atténué comprennent : une température de 37°C, une vitesse d'aération de 1 vvm (volume/volume minute) et un volume de germes de 5 % en volume/volume par rapport à la solution de milieu de culture, l'incubation étant effectuée à une vitesse d'agitation de 100 tr/min pendant les 2 premières heures et ensuite de 600 tr/min pendant les 10 à 20 heures suivantes, de préférence pendant 12 à 16 heures.

27. Agent thérapeutique pour traiter une infection par *Pseudomonas aeruginosa* chez un patient présentant cette infection, ledit agent comprenant au moins une immunoglobuline spécifique dirigée contre au moins une souche de *Pseudomonas aeruginosa*, dans lequel ladite immunoglobuline est induite par injection, à un hôte approprié, de protéines de parois cellulaires ayant un poids moléculaire compris entre 10 000 et 100 000, provenant au moins d'une souche atténuée de *Pseudomonas aeruginosa* choisie dans l'ensemble constitué par :
- souche de *Pseudomonas aeruginosa* ayant le numéro d'accès de la Fédération Coréenne de Collection de Cultures KCCM 10029 (CFCPA 10142),
- souche de *Pseudomonas aeruginosa* ayant le numéro d'accès de la Fédération Coréenne de Collection de Cultures KCCM 10030 (CFCPA 20215),
- souche de *Pseudomonas aeruginosa* ayant le numéro d'accès de la Fédération Coréenne de Collection de Cultures KCCM 10031 (CFCPA 30720),
- souche de *Pseudomonas aeruginosa* ayant le numéro d'accès de la Fédération Coréenne de Collection de Cultures KCCM 10032 (CFCPA 40057),
- souche de *Pseudomonas aeruginosa* ayant le numéro d'accès de la Fédération Coréenne de Collection de Cultures KCCM 10033 (CFCPA 50243),
- souche de *Pseudomonas aeruginosa* ayant le numéro d'accès de la Fédération Coréenne de Collection de Cultures KCCM 10034 (CFCPA 60534) et
- souche de *Pseudomonas aeruginosa* ayant le numéro d'accès de la Fédération Coréenne de Collection de Cultures KCCM 10035 (CFCPA 70018),
en une quantité suffisante pour induire une réponse immune chez ledit hôte pour induire la production de ladite immunoglobuline de façon au moins à atténuer les effets secondaires fâcheux de la maladie causée par *Pseudomonas aeruginosa*.

28. Agent thérapeutique selon la revendication 27, contenant une immunoglobuline dirigée contre chacune des souches de *Pseudomonas aeruginosa* correspondant aux immunotypes 1, 2, 3, 4, 5, 6 et 7 de Fischer.

29. Agent thérapeutique selon la revendication 27, contenant des immunoglobulines dirigées contre quatre souches quelconques de *Pseudomonas aeruginosa* correspondant aux immunotypes 1, 2, 3, 4, 5, 6 et 7 de Fischer.

30. Agent thérapeutique selon la revendication 27, contenant des immunoglobulines dirigées contre trois souches quelconques de *Pseudomonas aeruginosa* correspondant aux immunotypes 1, 2, 3, 4, 5, 6 et 7 de Fischer.

31. Agent thérapeutique selon la revendication 27 sous une forme lyophilisée.

32. Agent thérapeutique selon la revendication 31, comprenant, en outre, un véhicule pharmaceutiquement acceptable approprié choisi dans l'ensemble constitué par le mannitol, le lactose, le saccharose et l'albumine humaine.

33. Agent thérapeutique selon la revendication 27 à l'état liquide et comprenant, en outre, des véhicules pharmaceutiquement acceptables appropriés.

34. Agent thérapeutique selon la revendication 27, préparé sous une forme pharmaceutique dosée en tant que solution injectable, comprimé, capsule, collyre, produit à pulvériser ou onguent.
